# EUROPÄISCHE PATENTSCHRIFT

(11) **EP 1 339 716 B1**
(45) Veröffentlichungstag und Bekanntmachung des Hinweises auf die Patenterteilung: **03.11.2004**
(21) Anmeldenummer: 01997487.2
(22) Anmeldetag: 09.11.2001
(51) Int. Cl.: C07D 471/04, A61K 31/437, A61P 9/00, A61P 15/00, A61P 25/00

(54) **LACTAM-SUBSTITUIERTE PYRAZOLOPYRIDINDERIVATE**
LACTAM-SUBSTITUTED PYRAZOLOPYRIDINE DERIVATIVES
DERIVES DE PYRAZOLOPYRIDINE SUBSTITUES PAR UN LACTAME

(30) Priorität: 22.11.2000 DE 10057752; 11.05.2001 DE 10122895
(43) Veröffentlichungstag der Anmeldung: 03.09.2003
(73) Patentinhaber: Bayer HealthCare AG, 51368 Leverkusen (DE)
(72) Erfinder: STASCH, Johannes-Peter, 42651 Solingen (DE); FEURER, Achim, 69259 Wilhemsfeld (DE); WEIGAND, Stefan, 42115 Wuppertal (DE); STAHL, Elke, 51467 Bergisch Gladbach (DE); FLUBACHER, Dietmar, 79110 Freiburg (DE); ALONSO-ALIJA, Cristina, 42781 Haan (DE); WUNDER, Frank, 42117 Wuppertal (DE); LANG, Dieter, 42553 Velbert (DE); DEMBOWSKY, Klaus, Boston, MA 02116 (US); STRAUB, Alexander, 42117 Wuppertal (DE); PERZBORN, Elisabeth, 42327 Wuppertal (DE)
(86) Internationale Anmeldenummer: PCT/EP2001/012965
(87) Internationale Veröffentlichungsnummer: WO 2002/042299

(56) Entgegenhaltungen:
- WO-A-00/06568
- WO-A-00/06569

## Beschreibung

Die vorliegende Erfindung betrifft neue chemische Verbindungen, welche die lösliche Guanylatcyclase stimulieren, ihre Herstellung und ihre Verwendung als Arzneimittel, insbesondere als Arzneimittel zur Behandlung von Herz-Kreislauf-Erkrankungen.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriposphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch CO ist in der Lage, am Eisen-Zentralatom des Häms anzugreifen, wobei die Stimulierung durch CO deutlich geringer ist als die durch NO.

Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion und der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Atherosklerose, Angina pectoris, Herzinsuffizienz, Thrombosen, Schlaganfall und Myokardinfarkt fuhren kann.

Eine auf die Beeinflussung des cGMP-Signalweges in Organismen abzielende NOunabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriffe am Eisenzentralatom des Häms. Neben den Nebenwirkungen gehört die Toleranzentwicklung zu den entscheidenden Nachteilen dieser Behandlungsweise.

In den letzten Jahren wurden einige Substanzen beschrieben, die die lösliche Guanylatcyclase direkt, d.h. ohne vorherige Freisetzung von NO stimulieren, wie beispielsweise 3-(5'-Hydroxymethyl-2'-furyl)-1-benzylindazol (YC-1, Wu et al., Blood 84 (1994), 4226; Mülsch et al., Br.J.Pharmacol. 120 (1997), 681), Fettsäuren (Goldberg et al, J. Biol. Chem. 252 (1977), 1279), Diphenyliodonium-hexafluorophosphat (Pettibone et al., Eur. J. Pharmacol. 116 (1985), 307), Isoliquiritigenin (Yu et al., Brit. J. Pharmacol. 114 (1995), 1587) sowie verschiedene substituierte Pyrazolderivate (WO 98/16223).

Weiterhin sind in der WO 98/16507, WO 98/23619, WO 00/06567, WO 00/06568, WO 00/06569 und WO 00/21954 Pyrazolopyridinderivate als Stimulatoren der löslichen Guanylatcyclase beschrieben. In diesen Patentanmeldungen sind auch Pyrazolopyridine beschrieben, welche einen Pyrimidinrest in 3-Position aufweisen. Derartige Verbindungen weisen eine sehr hohe in vitro Aktivität bezüglich der Stimulation der löslichen Guanylatcyclase auf. Allerdings zeigte es sich, dass diese Verbindungen hinsichtlich ihrer in vivo-Eigenschaften wie beispielsweise ihrem Verhalten in der Leber, ihrem pharmakokinetischen Verhalten, ihrer Dosis-Wirkungsbeziehung oder ihrem Metabolisierungsweg einige Nachteile aufweisen.

Es war daher die Aufgabe der vorliegenden Erfindung, weitere Pyrazolopyridinderivate bereitzustellen, welche als Stimulatoren der löslichen Guanylatcyclase wirken, aber nicht die vorstehend aufgeführten Nachteile der Verbindungen aus dem Stand der Technik aufweisen.

Diese Aufgabe wird gemäß der vorliegenden Erfindungen durch Verbindungen gemäß Anspruch 1 gelöst. Diese neuen Pyrazolopyridinderivate zeichnen sich durch einen Pyrimidinrest in 3-Position aus, der ein bestimmtes Substitutionsmuster aufweist, nämlich einen cyclischen Lactamrest in 5-Position des Pyrimidinrings sowie gegebenenfalls eine Aminogruppe in 4-Position des Pyrimidinrings.

Im einzelnen betrifft die vorliegende Erfindung Verbindungen der Formel (I) 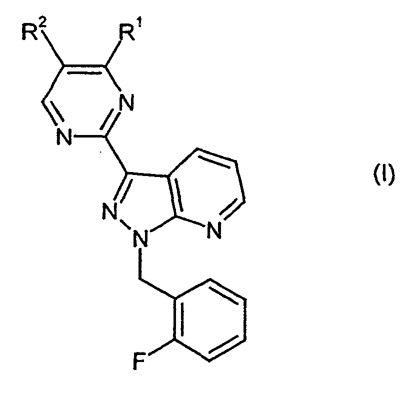 worin
- R¹: für NH₂ oder für NHCO-C₁₋₆-alkyl steht;
- R²: für einen Rest der Formel R³NCOR⁴ steht, der über das Stickstoffatom an den Rest des Moleküls gebunden ist,
wobei
R³ und R⁴ zusammen mit der Amidgruppe, an die sie gebunden sind, einen fünf- bis siebengliedrigen Heterocyclus bilden, der gesättigt oder teilweise ungesättigt sein kann, gegebenenfalls ein weiteres Heteroatom aus der Gruppe N, O, S enthalten kann und ein bis fünf weitere Substituenten aus der Gruppe Oxo, C₁₋₆-Alkyl, Hydroxy, Hydroxy-C₁₋₆-alkyl, Halogen aufweisen kann beziehungsweise an einen C₆₋₁₀-Arylring oder an einen C₃₋₈-Cycloalkylring, bei welchem gegebenenfalls zwei Kohlenstoffatome über ein Sauerstoffatom miteinander verbunden sind, anelliert sein kann;
sowie Salze, Isomere und Hydrate davon.

Gemäß einer alternativen Ausführungsform betrifft die vorliegende Erfindung Verbindungen der Formel (I), worin
- R¹: für NH₂ oder für NHCO-C₁₋₆-alkyl steht;
- R²: für einen Rest der Formel R³NCOR⁴ steht, der über das Stickstoffatom an den Rest des Moleküls gebunden ist,
wobei
R³ und R⁴ zusammen mit der Amidgruppe, an die sie gebunden sind, einen gesättigten fünf- bis siebengliedrigen Heterocyclus bilden, der gegebenenfalls ein weiteres Heteroatom aus der Gruppe N, O, S enthalten kann und ein bis fünf weitere Substituenten aus der Gruppe Oxo, C₁₋₄-Alkyl, Hydroxy, Hydroxy-C₁₋₄-alkyl, Halogen aufweisen kann beziehungsweise an einen C₆₋₁₀-Arylring oder an einen C₃₋₈-Cycloalkylring, bei welchem gegebenenfalls zwei Kohlenstoffatome über ein Sauerstoffatom miteinander verbunden sind, anelliert sein kann;
sowie Salze, Isomere und Hydrate davon.

Gemäß einer weiteren alternativen Ausführungsform betrifft die vorliegende Erfindung Verbindungen der Formel (I), worin
- R¹: für NH₂ oder für NHCOCH₃ steht;
- R²: für einen Rest der Formel R³NCOR⁴ steht, der über das Stickstoffatom an den Rest des Moleküls gebunden ist,
wobei
R³ und R⁴ zusammen mit der Amidgruppe, an die sie gebunden sind, einen gesättigten fünf- bis siebengliedrigen Heterocyclus bilden, der gegebenenfalls ein weiteres Heteroatom aus der Gruppe N, O, S enthalten kann und ein bis fünf weitere Substituenten aus der Gruppe Oxo, C₁₋₄-Alkyl aufweisen kann beziehungsweise an einen Phenylring oder an einen C₃₋₈-Cycloalkylring, bei welchem gegebenenfalls zwei Kohlenstoffatome über ein Sauerstoffatom miteinander verbunden sind, anelliert sein kann;
sowie Salze, Isomere und Hydrate davon.

Gemäß einer weiteren Ausführungsform betrifft die vorliegende Erfindung Verbindungen der Formel (I), worin
- R¹: für NH₂ steht;
- R²: für einen Rest der Formel R³NCOR⁴ steht, der über das Stickstoffatom an den Rest des Moleküls gebunden ist,
wobei
R³ und R⁴ zusammen mit der Amidgruppe, an die sie gebunden sind, einen fünf- bis siebengliedrigen Heterocyclus bilden, der gesättigt oder teilweise ungesättigt sein kann, gegebenenfalls ein weiteres Heteroatom aus der Gruppe N, O, S enthalten kann und ein bis fünf weitere Substituenten aus der Gruppe Oxo, C₁₋₆-Alkyl, Hydroxy, Hydroxy-C₁₋₆-alkyl, Halogen aufweisen kann beziehungsweise an einen C₆₋₁₀-Arylring anelliert sein kann;
sowie Salze, Isomere und Hydrate davon.

Gemäß einer weiteren Ausführungsform betrifft die vorliegende Erfindung Verbindungen der Formel (I), bei denen
- R¹: für NH₂ steht;
- R²: für einen Rest der Formel R³NCOR⁴ steht, der über das Stickstoffatom an den Rest des Moleküls gebunden ist,
wobei
R³ und R⁴ zusammen mit der Amidgruppe, an die sie gebunden sind, einen gesättigten fünf- bis siebengliedrigen Heterocyclus bilden, gegebenenfalls ein weiteres Sauerstoffatom enthalten kann und ein bis fünf weitere Substituenten aus der Gruppe Oxo, C₁₋₄-Alkyl, Hydroxy, Hydroxy-C₁₋₄-alkyl, F aufweisen kann beziehungsweise an einen C₆₋₁₀-Arylring anelliert sein kann;
sowie Salze, Isomere und Hydrate davon.

Gemäß einer weiteren Ausführungsform betrifft die vorliegende Erfindung Verbindungen der Formel (I), bei denen
- R¹: für NH₂ steht;
- R²: für einen Rest der Formel R³NCOR⁴ steht, der über das Stickstoffatom an den Rest des Moleküls gebunden ist,
wobei
R³ und R⁴ zusammen mit der Amidgruppe, an die sie gebunden sind, einen fünf- oder sechsgliedrigen gesättigten Heterocyclus bilden, der gegebenenfalls ein weiteres Sauerstoffatom enthalten kann und ein bis fünf weitere Substituenten aus der Gruppe Oxo, C₁₋₄-Alkyl aufweisen kann beziehungsweise an einen Phenylring anelliert sein kann;
sowie Salze, Isomere und Hydrate davon.

Die erfindungsgemäßen Verbindungen der allgemeinen Formel (I) können auch in Form ihrer Salze vorliegen. Im allgemeinen seien hier Salze mit organischen oder anorganischen Basen oder Säuren genannt

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, p-Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak, oder organischen Aminen wie beispielsweise Ethylamin, Di- bzw. Triethylamin, Di- bzw. Triethanolamin, Dicyclohexylamin, Dimethylaminoethanol, Arginin, Lysin oder Ethylendiamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren als auch deren jeweilige Mischungen. Die Racemformen lassen sich ebenso wie die Diastereomeren in bekannter Weise, beispielsweise durch chromatographische Trennung, in die stereoisomer einheitlichen Bestandteile trennen. In den erfindungsgemäßen Verbindungen vorhandene Doppelbindungen können in der cis- oder trans- Konfiguration (Z- oder E-Form) vorliegen.

Weiterhin können bestimmte Verbindungen in tautomeren Formen vorliegen. Dies ist dem Fachmann bekannt, und derartige Verbindungen sind ebenfalls vom Umfang der Erfindung umfasst.

Weiterhin können die erfindungsgemäßen Verbindungen in Form ihrer Hydrate vorkommen, wobei die Zahl der an das Molekül gebundenen Wassermoleküle von der jeweiligen erfindungsgemäßen Verbindung abhängt.

Im Rahmen der vorliegenden Erfindung haben die Substituenten soweit nicht anders angegeben im allgemeinen die folgende Bedeutung:
Alkyl steht im allgemeinen für einen geradkettigen oder verzweigten Kohlenwasserstoffrest mit 1 bis 20 Kohlenstoffatomen. Beispielsweise seien Methyl, Ethyl, Propyl, Isopropyl, Butyl, Isobutyl, Pentyl, Isopentyl, Hexyl, Isohexyl, Heptyl, Isoheptyl, Octyl und Isooctyl, Nonyl, Decyl, Dodeyl, Eicosyl genannt.
Alkylen steht im allgemeinen für eine geradkettige oder verzweigte Kohlenwasserstoffbrücke mit 1 bis 20 Kohlenstoffatomen. Beispielsweise seien Methylen, Ethylen, Propylen, -Methylethylen, -Methylethylen, -Ethylethylen, -Ethylethylen, Butylen, -Methylpropylen, -Methylpropylen, -Methylpropylen, -Ethylpropylen, -Ethylpropylen, -Ethylpropylen, Pentylen, Hexylen, Heptylen, Octylen, Nonylen, Decylen, Dodeylen und Eicosylen genannt.
Alkenyl steht im allgemeinen für einen geradkettigen oder verzweigten Kohlenwasserstoffrest mit 2 bis 20 Kohlenstoffatomen und einer oder mehreren, bevorzugt mit einer oder zwei Doppelbindungen. Beispielsweise seien Allyl, Propenyl, Isopropenyl, Butenyl, Isobutenyl, Pentenyl, Isopentenyl, Hexenyl, Isohexenyl, Heptenyl, Isoheptenyl, Octenyl, Isooctenyl genannt.
Alkinyl steht im allgemeinen für einen geradkettigen oder verzweigten Kohlenwasserstoffrest mit 2 bis 20 Kohlenstoffatomen und einer oder mehreren, bevorzugt mit einer oder zwei Dreifachbindungen. Beispielsweise seien Ethinyl, 2-Butinyl, 2-Pentinyl und 2-Hexinyl benannt.
Acyl steht im allgemeinen für geradkettiges oder verzweigtes Niedrigalkyl mit 1 bis 9 Kohlenstoffatomen, das über eine Carbonylgruppe gebunden ist. Beispielsweise seien genannt: Acetyl, Ethylcarbonyl, Propylcarbonyl, Isopropylcarbonyl, Butylcarbonyl und Isobutylcarbonyl.
Alkoxy steht im allgemeinen für einen über einen Sauerstoffatom gebundenen geradkettigen oder verzweigten Kohlenwasserstoffrest mit 1 bis 14 Kohlenstoffatomen. Beispielsweise seien Methoxy, Ethoxy, Propoxy, Isopropoxy, Butoxy, Isobutoxy, Pentoxy Isopentoxy, Hexoxy, Isohexoxy, Heptoxy, Isoheptoxy, Octoxy oder Isooctoxy genannt. Die Begriffe "Alkoxy" und "Alkyloxy" werden synonym verwendet.
Alkoxyalkyl steht im allgemeinen für einen Alkylrest mit bis zu 8 Kohlenstoffatomen, der durch einen Alkoxyrest mit bis zu 8 Kohlenstoffatomen substituiert ist.
Alkoxycarbonyl kann beispielsweise durch die Formel 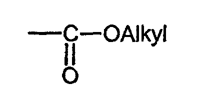 dargestellt werden.
Alkyl steht hierbei im allgemeinen für einen geradkettigen oder verzweigten Kohlenwasserstoffrest mit 1 bis 13 Kohlenstoffatomen. Beispielsweise seien die folgenden Alkoxycarbonylreste genannt: Methoxycarbonyl, Ethoxycarbonyl, Propoxycarbonyl, Isopropoxycarbonyl, Butoxycarbonyl oder Isobutoxycarbonyl.
Cycloalkyl steht im allgemeinen für einen cyclischen Kohlenwasserstoffrest mit 3 bis 8 Kohlenstoffatomen. Bevorzugt sind Cyclopropyl, Cyclopentyl und Cyclohexyl. Beispielsweise seien Cyclopentyl, Cyclohexyl, Cycloheptyl und Cyclooctyl genannt.
Cycloalkoxy steht im Rahmen der Erfindung für einen Alkoxyrest, dessen Kohlenwasserstoffrest ein Cycloalkylrest ist. Der Cycloalkylrest hat im allgemeinen bis zu 8 Kohlenstoffatome. Als Beispiele seien genannt: Cyclopropyloxy und Cyclohexyloxy. Die Begriffe "Cycloalkoxy" und "Cycloalkyloxy" werden synonym verwendet.
Aryl steht im allgemeinen für einen aromatischen Rest mit 6 bis 10 Kohlenstoffatomen. Bevorzugte Arylreste sind Phenyl und Naphthyl.
Halogen steht im Rahmen der Erfindung für Fluor, Chlor, Brom und Iod.
Heterocyclus steht im Rahmen der Erfindung im allgemeinen für einen gesättigten, ungesättigten oder aromatischen 3- bis 10-gliedrigen, beispielsweise 5- oder 6-gliedrigen Heterocyclus, der bis zu 3 Heteroatome aus der Reihe S, N und/oder O enthalten kann und der im Fall eines Stickstoffatoms auch über dieses gebunden sein kann. Beispielsweise seien genannt: Oxadiazolyl, Thiadiazolyl, Pyrazolyl, Pyridyl, Pyrimidinyl, Pyridazinyl, Pyrazinyl, Thienyl, Furyl, Pyrrolyl, Pyrrolidinyl, Piperazinyl, Tetrahydropyranyl, Tetrahydrofuranyl, 1,2,3 Triazolyl, Thiazolyl, Oxazolyl, Imidazolyl, Morpholinyl oder Piperidyl. Bevorzugt sind Thiazolyl, Furyl, Oxazolyl, Pyrazolyl, Triazolyl, Pyridyl, Pyrimidinyl, Pyridazinyl und Tetrahydropyranyl. Der Begriff "Heteroaryl" (bzw. "Hetaryl") steht für einen aromatischen heterocyclischen Rest.

Die erfindungsgemäßen Verbindungen der Formel (I) können hergestellt werden durch
[A] die Umsetzung der Verbindung der Formel (II) 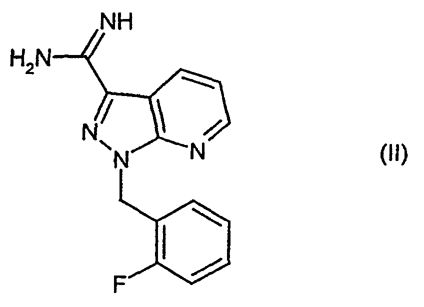 mit Verbindungen der Formel (III) 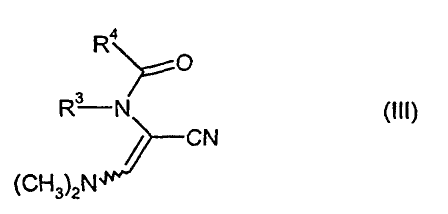 oder mit Verbindungen der Formel (IV) 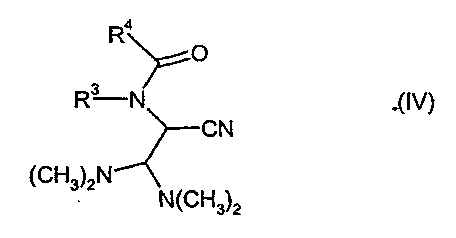 oder mit Verbindungen der Formel (V) 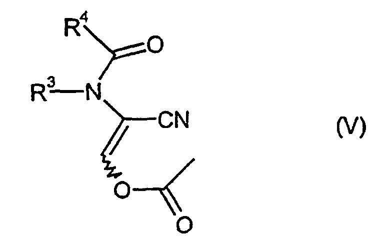 worin R³ und R⁴ die vorstehend angegebene Bedeutung haben,
in einem organischen Lösungsmittel gegebenenfalls in Gegenwart einer Base unter Erhitzen zu Verbindungen der Formel (I).

Die Verbindung der Formel (II) lässt sich gemäß folgendem Reaktionsschema herstellen:

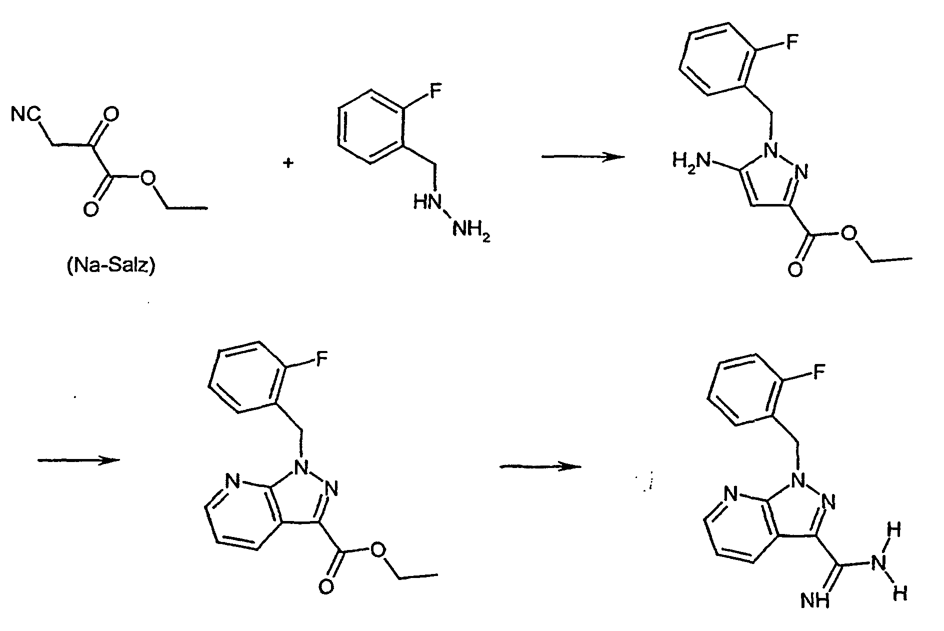

Die Verbindung der Formel (II) ist in einer mehrstufigen Synthese aus dem literaturbekannten Natriumsalz des Cyanobrenztraubensäureethylesters (Borsche und Manteuffel, Liebigs. Ann. Chem. 1934, 512, 97) erhältlich. Durch dessen Umsetzung mit 2-Fluorbenzylhydrazin unter Erhitzen und Schutzgasatmosphäre in einem inerten Lösungsmittel wie Dioxan erhält man den 5-Amino-1-(2-fluorbenzyl)-pyrazol-3-carbonsäureethylester, der durch Umsetzung mit Dimethylaminoacrolein im sauren Medium unter Schutzgasatmosphäre und Erhitzen zum entsprechenden Pyridinderivat cyclisiert. Dieses Pyridinderivat 1-(2-Fluorbenzyl)-1H-pyrazolo[3,4-b]pyridin-3-carbonsäureethylester wird durch eine mehrstufige Sequenz, bestehend aus Überführung des Esters mit Ammoniak in das entsprechende Amid, Dehydratisierung mit einem wasserentziehenden Mittel wie Trifluoressigsäureanhydrid zum entsprechenden Nitrilderivat, Umsetzung des Nitrilderivats mit Natriumethylat und abschließende Reaktion mit Ammoniumchlorid in die Verbindung der Formel (II) überführt.

Die Verbindungen der Formeln (III) und (IV) können aus den entsprechenden Lactamderivaten der Formel (VIII) 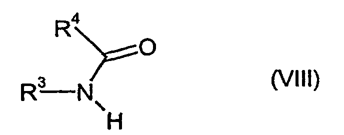 durch Umsetzung mit einem Halogenacetonitril wie beispielsweise Bromacetonitril, wobei das Acetonitrilderivat äquimolar oder im leichten Überschuss eingesetzt wird, in Gegenwart einer äquimolaren Menge oder eines leichten Überschusses einer Base wie beispielsweise einem Alkalimetallhydrid, insbesondere Natriumhydrid, in einem organischen Lösungsmittel wie beispielsweise einem cyclischen Ether, insbesondere Dioxan, oder vorzugsweise in einem Gemisch aus organischen Lösungsmitteln wie insbesondere einem Gemisch aus Dioxan und Dimethylformamid (DMF) im Verhältnis 3:1 bis 5:1, gegebenenfalls in Gegenwart einer äquimolaren Menge oder eines leichten Überschusses einer Lithiumverbindung wie Lithiumbromid vorzugsweise bei Normaldruck, Zusammengeben der Reaktanden unter Kühlung auf beispielsweise -5°C bis +5°C und anschließendem Rühren der Reaktionslösung für mehrere Stunden, beispielsweise 12 Stunden, bei erhöhter Temperatur, beispielsweise 50-80°C, vorzugsweise 60-70°C, zu den Verbindungen der Formel (IX) 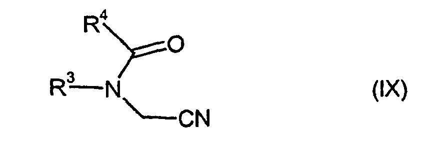 und anschließende Umsetzung der Verbindungen der Formel (IX) mit einer äquimolaren Menge oder eines leichten Überschusses an t-Butoxy-bis(dimethylamino)-methan (z.B. bei Aldrich käuflich erwerbbar) in einem organischen Lösungsmittel wie beispielsweise einem cyclischen Ether, insbesondere Dioxan, vorzugsweise bei Normaldruck und Rühren der Reaktionslösung für mehrere Stunden, beispielsweise 12 Stunden, bei erhöhter Temperatur, beispielsweise 50-80°C, vorzugsweise 60-70°C, hergestellt werden.

Die Verbindungen der Formel (VIII) sind z.B. von Aldrich oder Fluka erhältlich (-Butyrolactam, -Valerolactam, -Caprolactam, 5-Methyl-2-pyrrolidinon, Oxazolidin-2-on, 5,5-Dimethyl-1,3-oxazolidin-2,4-dion, 3-Methyl-2-pyrrolidinon) oder können nach Literaturvorschriften hergestellt werden. Beispielsweise kann die Synthese von 5,5-Dimethylpyrrolidin-2-on nach J. Org. Chem., 14, 1949, 605-625, J. Heterocycl. Chem. 23, 1986, 53-57 oder Tetrahedron Lett. , 35, 1994, 293-296 erfolgen. Die Darstellung von 3-Morpholinon ist beispielsweise in den Patenten US-5,349,045, US-3,966,766 und US-4,156,683 sowie in J.Amer.Chem.Soc. 61; 1939; 1575 beschrieben. 3,3,4,4-Tetramethyl-pyrrolidin-2-on kann nach Justus Liebigs Ann. Chem. 1977; 8-19 hergestellt werden. Substituierte 3-Morpholinone sind durch Reaktion von -Chlorcarbonsäurechloriden mit substituierten 2-Aminoethanolen gemäß Tetrahedron Lett. 1995, 36, 3821-3824 herstellbar. 4,4-Dimethyl-1,3-oxazolidin-2-on kann aus 2-Amino-2-methylpropanol und Diethylcarbonat nach Tetrahedron, 47,1991,2801-2820 hergestellt werden.

Die Verbindungen der Formel (V) können aus den Verbindungen der Formel (IX) durch Umsetzung mit einem Überschuss, beispielsweise einem 2- bis 3-fachen Überschuss eines Ameisensäurederivats wie beispielsweise einem Ameisensäureester wie Ameisensäureethylester in einem organischen Lösungsmittel wie beispielsweise einem cyclischen Ether, vorzugsweise Tetrahydrofuran (THF), in Gegenwart eines Überschusses, beispielsweise eines 2- bis 3-fachen Überschusses einer Base, beispielsweise einer Alkalimetallbase, vorzugsweise KOtBu, vorzugsweise bei Normaldruck und Rühren der Reaktionslösung für mehrere Stunden, beispielsweise 1 bis 2 Stunden, bei Raumtemperatur, und anschließender Reaktion mit einem der eingesetzten Menge an Base entsprechenden Menge an Essigsäure und anschließender Reaktion mit einem Essigsäurederivat wie Acetylchlorid oder Acetanhydrid unter Kühlung und anschließendem Rühren vorzugsweise bei Normaldruck für wenige Minuten bis zu mehreren Stunden, beispielsweise 30 Minuten bis 2 Stunden, bei Raumtemperatur erhalten werden.

Die Verbindungen der Formel (II) können mit Verbindungen der Formel (III) beziehungsweise (IV) beziehungsweise (V) in einem organischen Lösungsmittel wie beispielsweise einem Kohlenwasserstoff, vorzugsweise einem aromatischen Kohlenwasserstoff, insbesondere Xylol oder Toluol, gegebenenfalls in Gegenwart einer Base, beispielsweise einer organischen Base wie einem Amin, vorzugsweise Triethylamin, vorzugsweise bei Normaldruck und Rühren der Reaktionslösung für mehrere Stunden, beispielsweise 12 Stunden, bei erhöhter Temperatur, beispielsweise 80-130°C, vorzugsweise 100-130°C, insbesondere 120°C, zu den erfindungsgemäßen Verbindungen der Formel (I) umgesetzt werden. Die Reaktanden können hierbei je nach ihrer Beschaffenheit in äquimolaren Mengen eingesetzt werden, oder einer der Reaktanden wird in bis zu dreifachem Überschuss eingesetzt.

Die erfindungsgemäßen Verbindungen der allgemeinen Formel (I) zeigen ein nicht vorhersehbares, wertvolles pharmakologisches Wirkspektrum.

Die erfindungsgemäßen Verbindungen der allgemeinen Formel (I) führen zu einer Gefäßrelaxation, Thrombozytenaggregationshemmung und zu einer Blutdrucksenkung sowie zu einer Steigerung des koronaren Blutflusses. Diese Wirkungen sind über eine direkte Stimulation der löslichen Guanylatzyklase und einem intrazellulären cGMP-Anstieg vermittelt. Außerdem verstärken die erfindungsgemäßen Verbindungen der allgemeinen Formel (I) die Wirkung von Substanzen, die den cGMP-Spiegel steigern, wie beispielsweise EDRF (Endothelium derived relaxing factor), NO-Donatoren, Protoporphyrin IX, Arachidonsäure oder Phenylhydrazinderivate.

Sie können daher in Arzneimitteln zur Behandlung von kardiovaskulären Erkrankungen wie beispielsweise zur Behandlung des Bluthochdrucks und der Herzinsuffizienz, stabiler und instabiler Angina pectoris, peripheren und kardialen Gefäßerkrankungen, von Arrhythmien, zur Behandlung von thromboembolischen Erkrankungen und Ischämien wie Myokardinfarkt, Himschlag, transistorisch und ischämische Attacken, periphere Durchblutungßtörungen, Verhinderung von Restenosen wie nach Thrombolysetherapien, percutan transluminalen Angioplastien (PTA), percutan transluminalen Koronarangioplastien (PTCA), Bypaß sowie zur Behandlung von Arteriosklerose, asthmatischen Erkrankungen und Krankheiten des Urogenitalsystems wie beispielsweise Prostatahypertrophie, erektile Dysfunktion, weibliche sexuelle Dysfunktion, Osteoporose, Gastroparese und Inkontinenz eingesetzt werden.

Die in der vorliegenden Erfindung beschriebenen Verbindungen der allgemeinen Formel (I) stellen auch Wirkstoffe zur Bekämpfung von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lernleistung, oder Gedächtnisleistung nach kognitiven Störungen, wie sie insbesondere bei Situationen/Krankheiten/Syndromen auftreten wie "Mild cognitive impairment", Altersassoziierte Lern- und Gedächtnisstörungen, Altersassozüerte Gedächtnisverluste, Vaskuläre Demenz, Schädel-Hirn-Trauma, Schlaganfall, Demenz, die nach Schlaganfällen auftritt ("post stroke dementia"), posttraumatisches Schädel Hirn Trauma, allgemeine Konzentrationsstörungen, Konzentrationsstörungen in Kindern mit Lern- und Gedächtnisproblemen, Alzheimersche Krankheit, Vaskuläre Demenz, Demenz mit Lewy-Körperchen, Demenz mit Degeneration der Frontallappen einschließlich des Pick's Syndroms, Parkinsonsche Krankheit, Progressive nuclear palsy, Demenz mit corticobasaler Degeneration, Amyolateralsklerose (ALS), Huntingtonsche Krankheit, Multiple Sklerose, Thalamische Degeneration, Creutzfeld-Jacob-Demenz, HIV-Demenz, Schizophrenie mit Demenz oder Korsakoff-Psychose. Sie eignen sich auch zur Behandlung von Erkrankungen des Zentralnervensystems wie Angst-, Spannungs- und Depressionszuständen, zentralnervös bedingten Sexualdysfunktionen und Schlafstörungen, sowie zur Regulierung krankhafter Störungen der Nahrungs-, Genuss- und Suchtmittelaufnahme.

Weiterhin eignen sich die Wirkstoffe auch zur Regulation der cerebralen Durchblutung und stellen somit wirkungsvolle Mittel zur Bekämpfung von Migräne dar.

Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen der allgemeinen Formel (I) zur Bekämpfung von Schmerzzuständen eingesetzt werden.

Zudem besitzen die erfindungsgemäßen Verbindungen antiinflammatorische Wirkung und können daher als entzündungshemmende Mittel eingesetzt werden.

Darüber hinaus umfasst die Erfindung die Kombination der erfindungsgemäßen Verbindungen der allgemeinen Formel (I) mit organischen Nitraten und NO-Donatoren.

Organische Nitrate und NO-Donatoren im Rahmen der Erfindung sind im allgemeinen Substanzen, die über die Freisetzung von NO bzw. NO-Species ihre therapeutische Wirkung entfalten. Bevorzugt sind Natriumnitroprussid, Nitroglycerin, Isosorbiddinitrat, Isosorbidmononitrat, Molsidomin und SIN-1.

Außerdem umfasst die Erfindung die Kombination mit Verbindungen, die den Abbau von cyclischem Guanosinmonophosphat (cGMP) inhibieren. Dies sind insbesondere Inhibitoren der Phosphodiesterasen 1, 2 und 5; Nomenklatur nach Beavo und Reifsnyder (1990) TiPS 11 S. 150 bis 155. Durch diese Inhibitoren wird die Wirkung der erfindungsgemäßen Verbindung potenziert und der gewünschte pharmakologische Effekt gesteigert.

### Biologische Untersuchungen

### Gefäßrelaxierende Wirkung in vitro

Kaninchen werden durch Nackenschlag betäubt und entblutet. Die Aorta wird entnommen, von anhaftendem Gewebe befreit, in 1,5 mm breite Ringe geteilt und einzeln unter einer Vorspannung in 5 ml-Organbäder mit 37 C warmer, carbogenbegaster Krebs Henseleit-Lösung folgender Zusammensetzung (mM) gebracht: NaCl: 119; KCl: 4,8; CaCl₂ x 2 H₂O: 1; MgSO₄ x 7 H₂O; 1,4; KH₂PO₄: 1,2; NaHCO₃:25; Glucose: 10. Die Kontraktionskraft wird mit Statham UC2-Zellen erfasst, verstärkt und über A/D-Wandler (DAS-1802 HC, Keithley Instruments München) digitalisiert sowie parallel auf Linienschreiber registriert. Zur Erzeugung einer Kontraktion wird Phenylephrin dem Bad kumulativ in ansteigender Konzentration zugesetzt. Nach mehreren Kontrollzyklen wird die zu untersuchende Substanz in jedem weiteren Durchgang in jeweils steigender Dosierung untersucht und die Höhe der Kontraktion mit der Höhe der im letzten Vordurchgang erreichten Kontraktion verglichen. Daraus wird die Konzentration errechnet, die erforderlich ist, um die Höhe des Kontrollwertes um 50 % zu reduzieren (IC₅₀). Das Standardapplikationsvolumen beträgt 5 µl, der DMSO-Anteil in der Badlösung entspricht 0,1 %. Die Ergebnisse sind nachstehend in Tabelle 1 aufgeführt:

**Tabelle 1:**

| **Gefäßrelaxierende Wirkung in vitro** | |
|---|---|
| Beispiel Nr. | IC₅₀ [µM] |
| 2 | 1,1 |
| 3 | 1,99 |
| 5 | 1,05 |
| 9 | 0,70 |
| 13 | 0,69 |
| 14 | 0,42 |
| 15 | 1,2 |
| 16 | 1,23 |
| 19 | 0,41 |
| 21 | 0,25 |
| 22 | 0,60 |

### Bestimmung der Leberclearance in vitro

Ratten werden anästhesiert, heparinisiert, und die Leber in situ über die Pfortader perfundiert. Ex vivo werden dann aus der Leber mittels Collagenase-Lösung die primären Ratten-Hepatozyten gewonnen. Es wurden 2^{·}10⁶ Hepatozyten pro ml mit jeweils der gleichen Konzentration der zu untersuchenden Verbindung bei 37°C inkubiert. Die Abnahme des zu untersuchenden Substrates über die Zeit wurde bioanalytisch (HPLC/UV, HPLC/Fluoreszenz oder LC/MSMS) an jeweils 5 Zeitpunkten im Zeitraum von 0-15 min nach Inkubationsstart bestimmt. Daraus wurde über Zellzahl und Lebergewicht die Clearance errechnet.

### Bestimmung der Plasmaclearance in vivo

Die zu untersuchende Substanz wird Ratten über die Schwanzvene intravenös als Lösung appliziert. Zu festgelegten Zeitpunkten wird den Ratten Blut entnommen, dieses wird heparinisiert und durch herkömmliche Maßnahmen Plasma daraus gewonnen. Die Substanz wird im Plasma bioanalytisch quantifiziert. Aus den so ermittelten Plasmakonzentrations-Zeit-Verläufen werden über herkömmliche hierfür verwendete nicht-kompartimentelle Methoden die pharmakokinetischen Parameter errechnet.

Zur vorliegenden Erfindung gehören pharmazeutische Zubereitungen, die neben nichttoxischen, inerten pharmazeutisch geeigneten Trägerstoffen die erfindungsgemäßen Verbindungen der allgemeinen Formel (I) enthält sowie Verfahren zur Herstellung dieser Zubereitungen.

Die Wirkstoff können gegebenenfalls in einem oder mehreren der oben angegebenen Trägerstoffe auch in mikroverkapselter Form vorliegen.

Die therapeutisch wirksamen Verbindungen der allgemeinen Formel (I) sollen in den oben aufgeführten pharmazeutischen Zubereitungen in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen der allgemeinen Formel (I) auch weitere pharmazeutische Wirkstoffe enthalten.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,01 bis etwa 700, vorzugsweise 0,01 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 0,1 bis etwa 80, insbesondere 0,1 bis 30 mg/kg Körpergewicht.

Die vorliegende Erfindung wird nachstehend anhand von nicht einschränkenden bevorzugten Beispielen näher dargestellt. Soweit nicht anderweitig angegeben, beziehen sich alle Mengenangaben auf Gewichtsprozente.

### Beispiele

### Abkürzungen:

- RT:: Raumtemperatur
- EE:: Essigsäureethylester
- DMF:: N,N-Dimethylformamid

### Methoden zur Ermittlung der HPLC-Retentionszeitenn bzw. präparative Trennmethoden:

*Methode A* (*HPLC-MS*)*:*
- Eluent:: A= CH₃CN B=0.6 g 30 %ige HCl/l H₂O
- Fluss:: 0.6 ml/min
- Säulenofen:: 50°C
- Säule:: Symmetry C18 2.1*150mm

| Gradient: | | | |
|---|---|---|---|
| Zeit (min) | %A | %B | Fluss (ml/min) |
| 0 | 10 | 90 | 0.6 |
| 4 | 90 | 10 | 0.6 |
| 9 | 90 | 10 | 0.8 |

*Methode B* (*HPLC*)*:*
- Eluent:: A=5 ml HClO₄/l H₂O, B=CH₃CN
- Fluss:: 0.75 ml/min
- L-R Temperatur:: 30.00°C 29.99°C
- Säule:: Kronasil C18 60*2mm

| Gradient: | | |
|---|---|---|
| Zeit (min) | %A | %B |
| 0.50 | 98 | 2 |
| 4.50 | 10 | 90 |
| 6.50 | 10 | 90 |
| 6.70 | 98 | 2 |
| 7.50 | 98 | 2 |

*Methode C* (*HPLC*)*:*
- Eluent:: A= H₃PO₄ 0.01 mol/l, B=CH₃CN
- Fluss:: 0.75 ml/min
- L-R Temperatur:: 30.01°C 29.98°C
- Säule:: Kromasil C18 60*2mm

| Gradient: | | |
|---|---|---|
| Zeit (min) | %A | %B |
| 0.00 | 90 | 10 |
| 0.50 | 90 | 10 |
| 4.50 | 10 | 90 |
| 8.00 | 10 | 90 |
| 8.50 | 90 | 10 |
| 10.00 | 90 | 10 |

*Methode D (chirale HPLC*)*:*
- Eluent:: 50 % iso-Hexan, 50 % Ethanol
- Fluss:: 1.00 ml/min
- Temperatur:: 40°C
- Säule:: 250*4,6 mm, gefüllt mit Chiralcel OD, 10 µm

*Methode E* (*HPLC-MS*)*:*
- Eluent:: A= CH₃CN E=0.3 g 30 %ige HCl/l H₂O
- Fluss:: 0.9 ml/min
- Säulenofen:: 50°C
- Säule:: Symmetry C18 2.1*150mm

| Gradient: | | | |
|---|---|---|---|
| Zeit (min) | %A | %B | Fluss (ml/min) |
| 0 | 10 | 90 | 0.9 |
| 3 | 90 | 10 | 1.2 |
| 6 | 90 | 10 | 1.2 |

*Methode F* (*präparative HPLC*)*:*
- Eluent:: A = Milli-Q-Wasser, B = Acetonitril, C = 1 %ige Trifluoressigsäure
- Fluss:: 25 ml/min
- Temperatur:: 50°C
- Packungsmaterial:: Kromasil 100 C 185 µm 250x20 mm Nr. 1011314R

| Zeit (min) | A | B | C |
|---|---|---|---|
| 0 | 72 | 10 | 18 |
| 30 | 32 | 60 | 8 |
| 30.1 | 4 | 95 | 1 |
| 40 | 4 | 95 | 1 |
| 48 | 72 | 10 | 18 |

*Methode G = (LC-MS):*
- Eluent:: A = Acetonitril + 0.1 % Ameisensäure, B = Wasser + 0.1 % Ameisensäure
- Fluss:: 25 ml/min
- Temperatur:: 40°C
- Packungsmaterial:: Symmetry C 18, 50x2.1 mm, 3.5 µm.

| Zeit (min) | A | B |
|---|---|---|
| 0 | 10 | 90 |
| 4.0 | 90 | 10 |
| 6.0 | 90 | 10 |
| 6.1 | 10 | 90 |
| 7.5 | 10 | 90 |

*Methode H* (*GC-MS*)*:*
- Trägergas:: Helium
- Fluss:: 1.5 ml/min
- Anfangstemperatur:: 60°C
- Temperaturgradient:: 14°C/min bis 300°C, dann 1 min konst. 300°C
- Säule:: HP-5 30m x 320µm x 0.25µm (Filmdicke)
- Anfangszeit:: 2 min
- Frontinjektor-Temp.:: 250°C

### Ausgangsverbindungen:

### I. Synthese von Lactam-substituierten Acetonitrilen

### Beispiel I a: (3-Oxo-4-morpholinyl)acetonitril

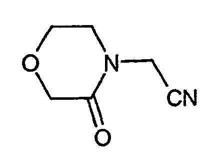

Zu 12,1 g (120 mmol) Morpholinon in einem Gemisch aus 150 ml Dioxan und 30ml DMF wurden unter Eiskühlung portionsweise 3,01 g (126 mmol) Natriumhydrid zugegeben und 40 min bei 0°C gerührt. Anschließend wurden 11,4 g (132 mmol) wasserfreies Lithiumbromid zugefügt und 30min. lang bei RT gerührt. Nach Zutropfen von 15,8 g (132 mmol) Bromacetonitril wurde über Nacht bei 65°C gerührt. Die Suspension wurde auf RT abgekühlt und auf gesättigte NaCl-Lsg. gegossen. Extraktion mit Ethylacetat, Trocknen der organischen Phase über Natriumsulfat und Abdestillieren des Lösungsmittels am Rotationsverdampfer und Chromatographie des Rückstands an Kieselgel mit Dichlormethan/Methanol 30:1 lieferte noch 33 % DMF enthaltende Produkt, das ohne weitere Reinigung in die nächste Reaktion eingesetzt wurde.
- Ausbeute:: 13.0 g (52 %, bereinigt um DMF-Gehalt)
- ¹H-NMR:: (300 MHz, D₆-DMSO), δ = 3.45 (t, 2H, CH₂N), 3.88 (t, 2H, CH₂O), 4.12 (s, 2H, CH₂O), 4.4 (s, 2H, CH₂CN)
- MS:: (ESI pos.), *m*/*z* = 141 ([M+H]⁺), 182 ([M+H+CH₃CN]⁺)

Auf analoge Weise wurden aus den entsprechenden Lactamen bzw. aus den entsprechenden cyclischen Carbamaten durch Umsetzung mit Bromacetonitril hergestellt:

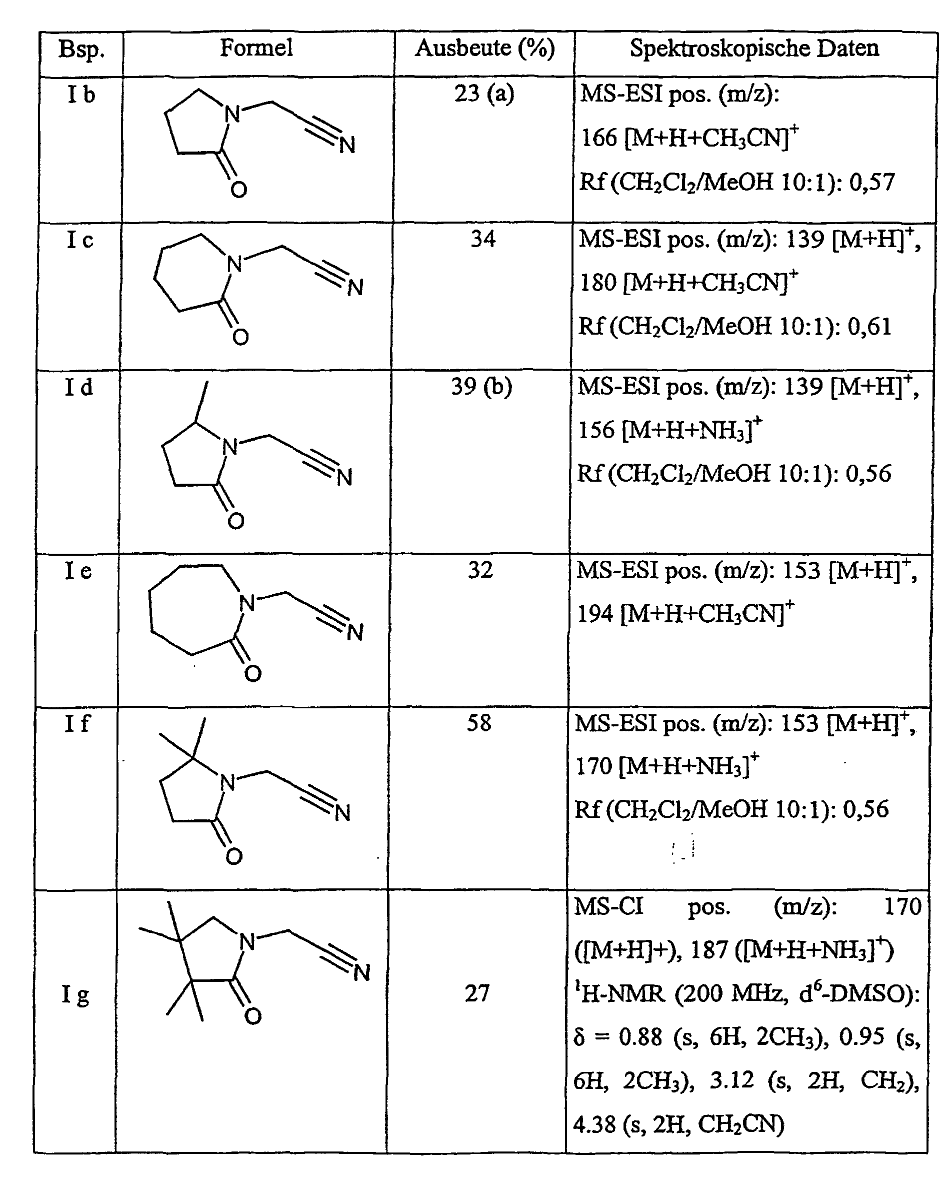

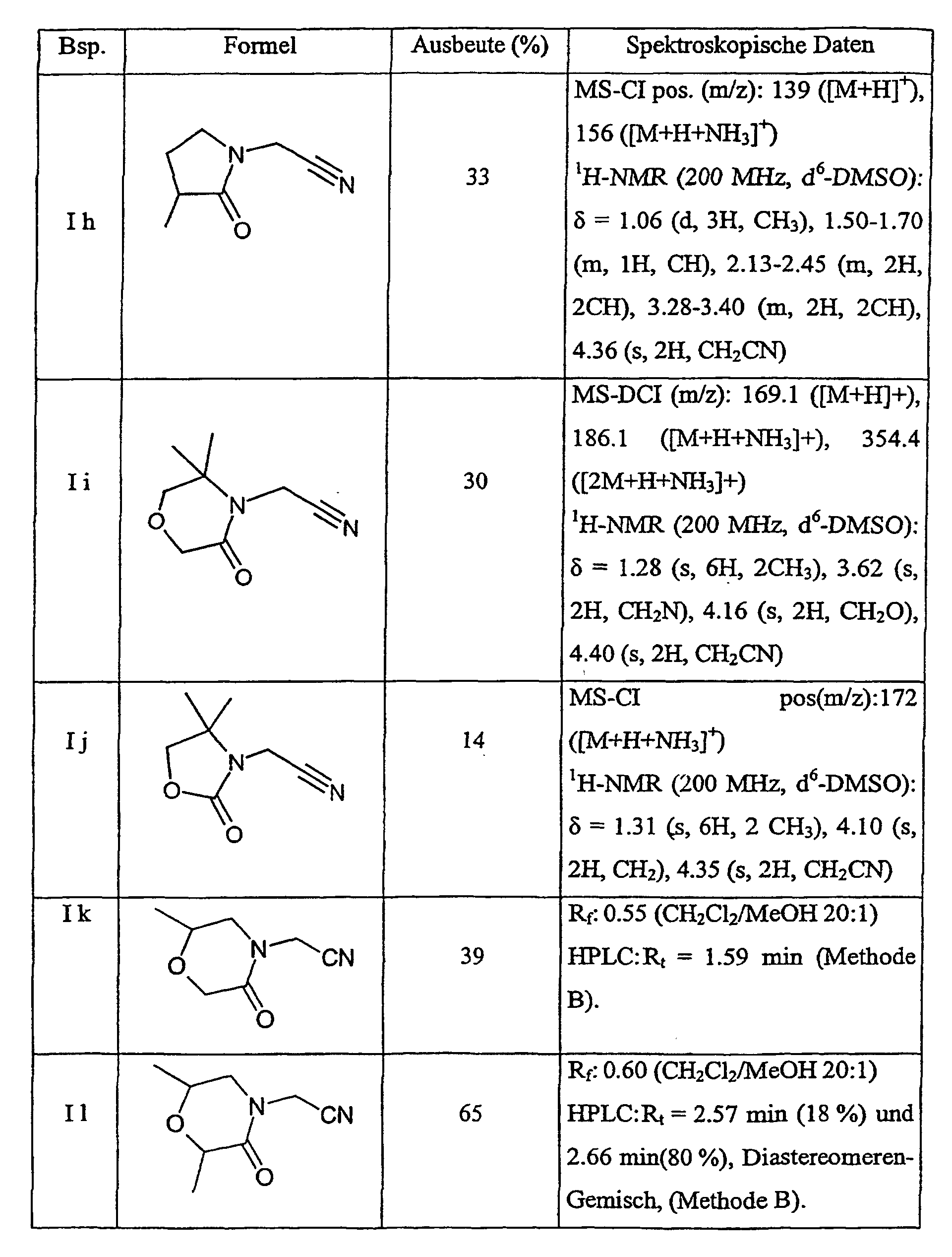

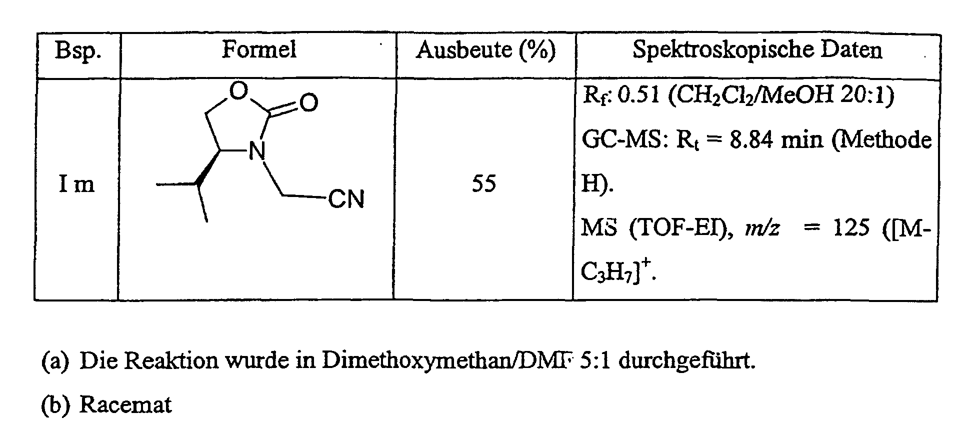

### II. Synthese von Lactam-substituierten Acryl- bzw. Propionitrilen

### Beispiel II a: 3-(Dimethylamino)-2-(3-oxo-4-morpholinyl)-2 propen-nitril (E/Z-Gemisch)

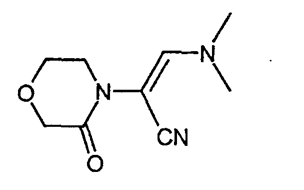

Eine Lösung aus 13.0 g (67 %ig in DMF, 62.2 mmol) (3-Oxo-4-morpholinyl)acetonitril aus Bsp. I a in 150 ml Dioxan wurde bei Raumtemperatur mit 16.2 g (19.2 ml, 92.8 mmol) tert-Butoxy-bis(dimethylamino)methan versetzt und die Mischung anschließend bei 80°C über Nacht gerührt. Der nach Abziehen des Lösungsmittels am Rotationsverdampfer verbleibende Rückstand wurde an Kieselgel mit Dichlormethan/Methanol 20:1 chromatographiert. Es wurde ein braunes Öl erhalten, aus dem Produkt auskristallisierte. Nach Behandeln mit Methanol im Ultraschallbad wurde Cyclohexan zugegeben, um das Produkt als hellbraunen Feststoff auszufällen. Das Produkt liegt laut NMR-Spektrum (300 MHz, D₆-DMSO) als E/Z-Gemisch vor.
- Ausbeute:: 10.2 g (84 %)
- ¹H-NMR:: (200 MHz, D₆-DMSO), δ = 2.88 und 3.03 (2s, zusammen 6H, N(CH₃)₂),
3.38-3.52 (m, 2H, CH₂N), 3.80-3.91 (m, 2H, CH₂O), 4.09 und 4.12 (2s, zusammen 2H, CH₂O), 6.91 und 7.01 (2s, zusammen 1H, Olefin-CH)
- MS:: (ESI pos.), *m*/*z* = 196 ([M+H]⁺)

Auf analoge Weise wurden erhalten:

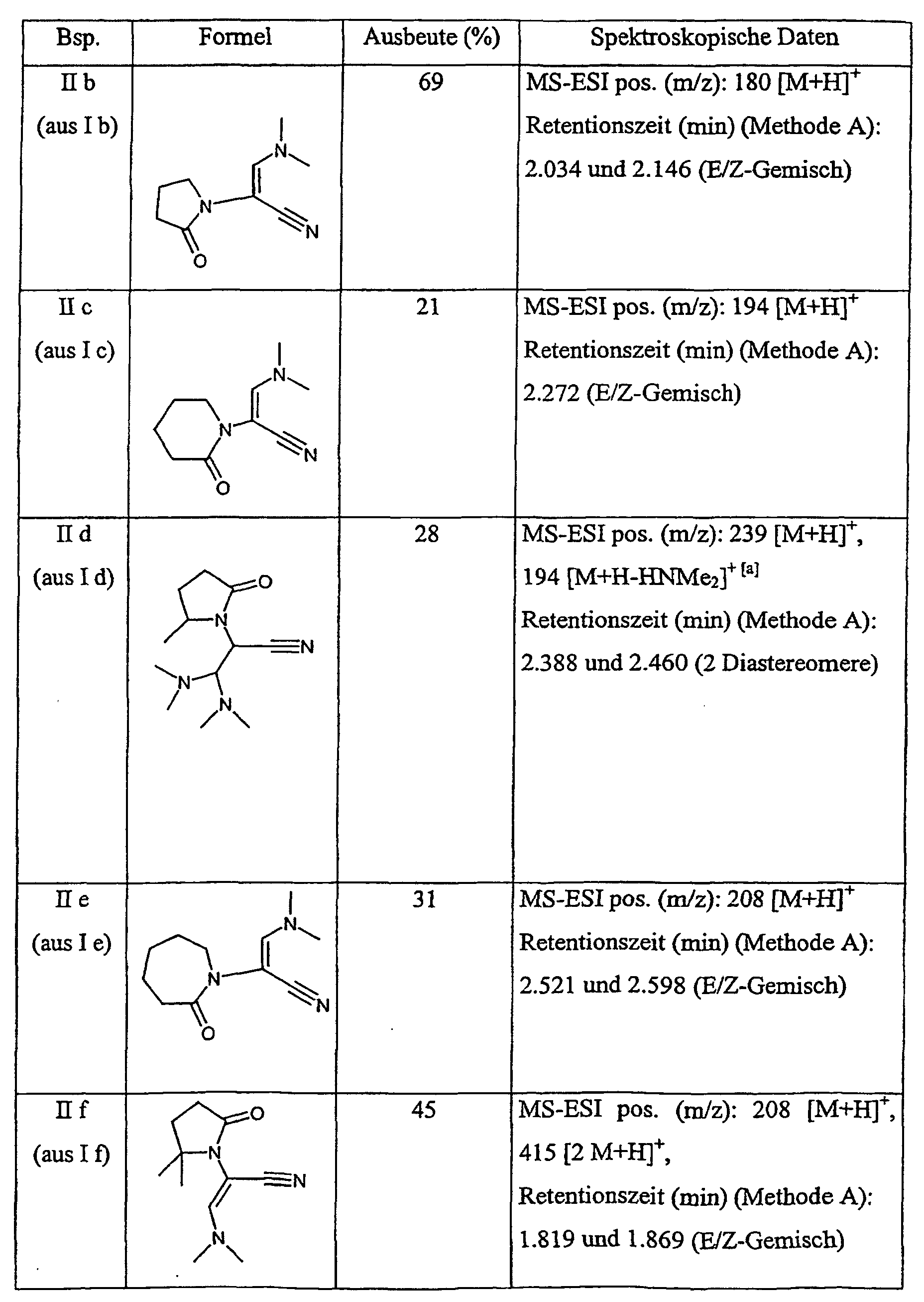

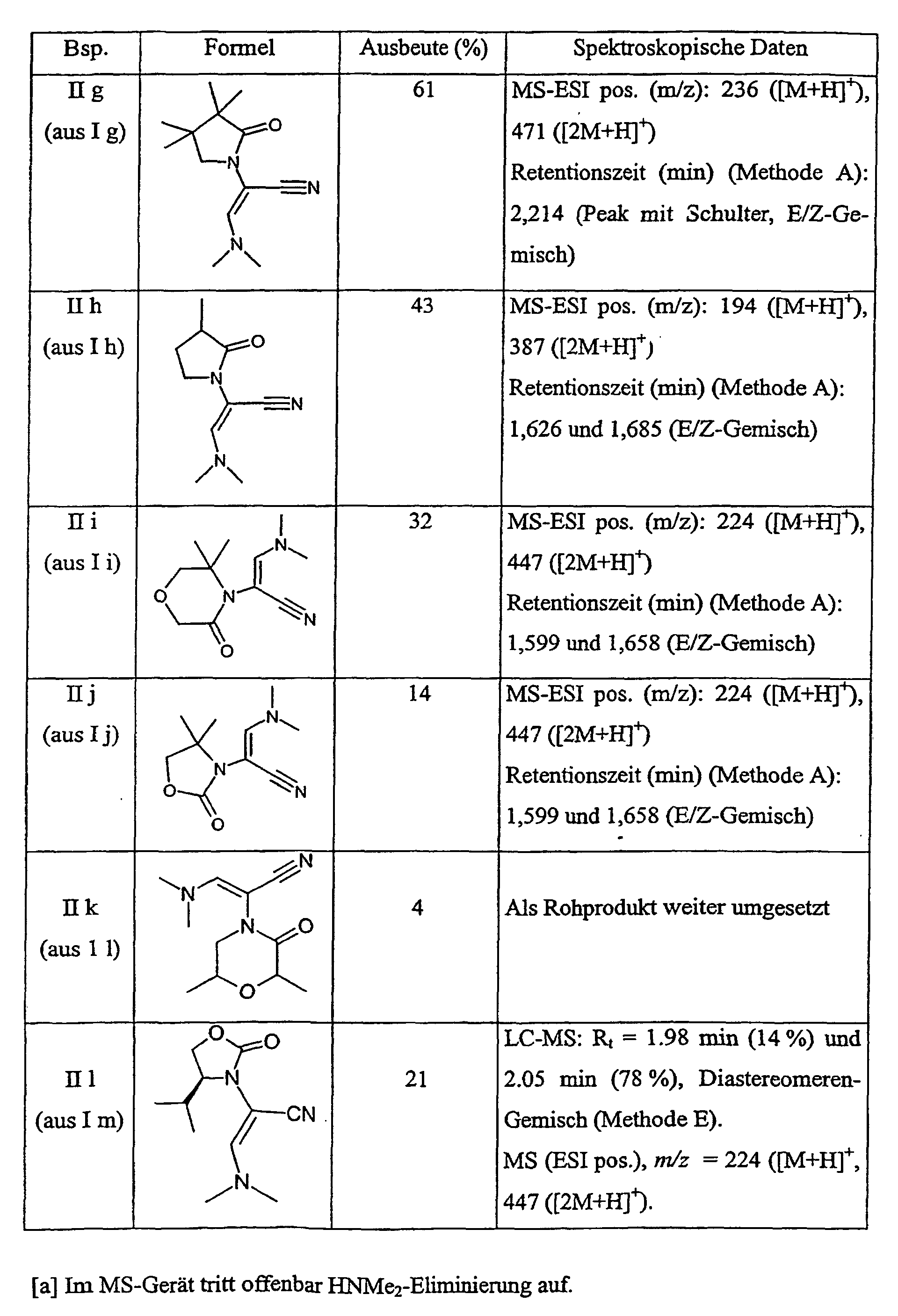

### III. Synthese von 3-(Dimethylamino)-(5,5-dimethyl-2,4-dioxo-1,3-oxazolidin-3-yl)-2-propennitril

### III a) (5,5-Dimethyl-2,4-dioxo-1,3-oxazolidin-3-yl)acetonitril

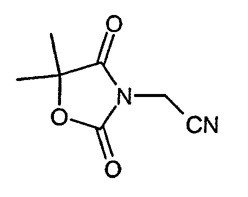

Eine Lösung von 10.0 g (77.5 mmol) 5,5-Dimethyloxazolidin-2,4-dion in 30 ml DMF wurde unter Eiskühlung zu einer Suspension von 3.25 g (81.3 mmol) Natriumhydrid (60 %ig in Mineralöl) in 200 ml Dioxan getropft. Die Mischung wurde 40 min gerührt und dabei auf Raumtemperatur erwärmen gelassen. Anschließend wurden 7,40 g (85.2 mmol) wasserfreies Lithiumbromid zugefügt und 20 min lang bei RT gerührt. Nach Zutropfen von 10.2 g (85.2 mmol) Bromacetonitril wurde über Nacht bei 65°C gerührt. Die Suspension wurde auf RT abgekühlt und auf gesättigte NaCl-Lösung gegossen. Die organische Phase wurde auf Kieselgel aufgezogen und mit Dichlormethan/Methanol 50:1 chromatographiert. Die Produkt enthaltenden Fraktionen wurden zur teilweisen Entfernung von noch enthaltenem DMF am Hochvakuum getrocknet. Das so behandelte Produkt wies noch einen Anteil von 44 Mol-% DMF auf und wurde ohne weitere Reinigung in die nächste Reaktion eingesetzt.
- Ausbeute:: 16.0 g (69 %)
- ¹H-NMR:: (300 MHz, D₆-DMSO), δ = 1.52 (s, 6H, CH₃), 4.61 (s, 2H, CH₂CN)
- R_{f}-Wert:: 0.76 (Dichlormethan/Methanol 20:1)

### III b) 3-(Dimethylamino)-(5,5-dimethyl-2,4-dioxo-1,3-oxazolidin-3-yl)-2-propennitril (E/Z-Gemisch)

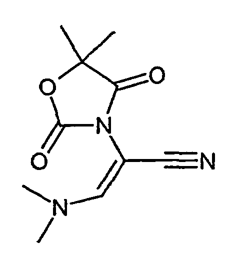

Eine Lösung aus 10.0 g (56 %ig in DMF, 33.3 mmol) (5,5-Dimethyl-2,4-dioxo-1,3-oxazolidin-3-yl)acetonitril aus Beispiel III a in 150 ml Dioxan wurde bei Raumtemperatur mit 5.80 g (6.88 ml, 33.3 mmol) tert-Butoxy-bis(dimethylamino)methan versetzt und die Mischung anschließend bei 80°C über Nacht gerührt. Das Gemisch wurde auf Kieselgel aufgezogen und mit Cyclohexan/Ethylacetat-Gradient (1:1, 1:2, 1:3) chromatographiert. Die Produkt enthaltenden Fraktionen wurden eingeengt, in Ethylacetat aufgenommen und mit Cyclohexan ausgefällt. Der Niederschlag wurde abgesaugt und mit Diethylether gewaschen, wobei ein hellgelber Feststoff entstand Das Produkt liegt laut NMR-Spektrum (300 MHz, D₆-DMSO) als E/Z-Gemisch vor.
- Ausbeute:: 2.44 g (57 %)
- ¹H-NMR:: (200 MHz, D₆-DMSO), δ = 1.54 (s, 6H, CH₃), 3.11 und 3.32 (2s, zusammen 6H, N(CH₃)₂), 7.27 und 7.37 (2s, zusammen 1H, Olefin-CH)
- MS:: (ESI pos.), *m*/*z* = 224 ([M+H]⁺), 265 ([M+H+CH₃CN]⁺)

### IV. Synthese von 3-(Dimethylamino)-2-(2-oxo-1,3-oxazolidin-3-yl)-2-propennitril

### IV a) (2-Oxo-1,3-oxazolidin-3-yl)acetonitril

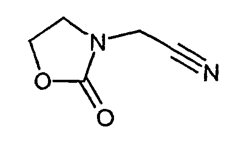

Eine Lösung von 15.0 g (172.3 mmol) 2-Oxazolidinon in 30 ml DMF wurde unter Eiskühlung zu einer Suspension von 7.23 g (180.9 mmol) Natriumhydrid (60 %ig in Mineralöl) in 200 ml Dioxan getropft. Die Mischung wurde 40 min gerührt und dabei auf Raumtemperatur erwärmen gelassen. Anschließend wurden 16.5 g (189.5 mmol) wasserfreies Lithiumbromid zugefügt und 40 min lang bei RT gerührt. Nach Zutropfen von 22.7 g (189.5 mmol) Bromacetonitril wurde über Nacht bei 65°C gerührt. Die Suspension wurde aufRT abgekühlt und auf ges. NaCl-Lsg. gegossen. Die organische Phase wurde auf Kieselgel aufgezogen und mit Dichlormethan/Methanol 50:1 chromatographiert. Die Produkt enthaltenden Fraktionen wurden einer weiteren Chromatographie an Kieselgel unterzogen, wobei Cyclohexan/Ethylacetat 1:1 als Eluens verwendet wurde.
- Ausbeute:: 19.9 g (91 %)
- ¹H-NMR:: (300 MHz, D₆-DMSO), δ = 3.61 (t, 2H, CH₂N), 4.35 (t, 2H, CH₂O), 4.39 (s, 2H, CH₂CN)
- R_{f}-Wert:: 0.56 (Dichlormethan/Methanol 20:1)
- MS:: (EI), *m*/*z* (%) = 126 (60, M⁺), 67 (100)

### IV b) 3-(Dimethylamino)-2-(2-oxo-1,3-oxazolidin-3-yl)-2-propen-nitril (E/Z-Gemisch)

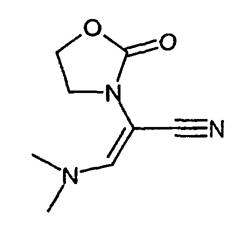

Eine Lösung aus 10.0 g (79.3 mmol) (2-Oxo-1,3-oxazolidin-3-yl)acetonitril aus Bsp. IV a in 200 ml Dioxan wurde bei Raumtemperatur mit 13.8 g (16.4 ml, 79.3 mmol) tert-Butoxy-bis(dimethylamino)methan versetzt und die Mischung anschließend 6 h bei 80°C und über Nacht bei Raumtemperatur gerührt. Die Mischung wurde am Rotationsverdampfer zur Trockne eingeengt und über Kieselgel mit Cyclohexan/Ethylacetat-Gradient (1:1, 1:2, 1:3) chromatographiert. Das Produkt liegt laut NMR-Spektrum (300 MHz, D₆-DMSO) als E/Z-Gemisch vor.
- Ausbeute:: 12.3 g (67 %)
- ¹H-NMR:: (200 MHz, D₆-DMSO), δ = 2.94 und 3.03 (2s, zusammen 6H, N(CH₃)₂),
3.58-3.68 (m, zusammen 2H,CH₂N), 4.23-4.42 (m, zusammen 2H,CH₂O),
6.97 und 7.13 (2s, zusammen 1H, Olefin-CH)
- MS:: (DCI.), *m*/*z* = 182 ([M+H]⁺), 199 ([M+H+NH₃]⁺, 380 ([2M+H+NH₃]⁺)

### V. Synthese von 1-(2-Fluorbenzyl)1H-pyrazolo[3,4-b]pyridin-3-carboxamidin

### V a) 5-Amino-1-(2-fluorbenzyl)-pyrazol-3-carbonsäureethylester

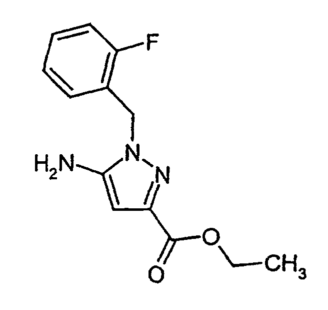

100 g (0.613 mol) Natriumsalz des Cyanobrenztraubensäureethylester (Darstellung analog Borsche und Manteuffel, Liebigs Ann. 1934, *512,* 97) werden unter gutem Rühren unter Argon in 2.5 1 Dioxan bei Raumtemperatur mit 111.75 g (75 ml, 0.98 mol) Trifluoressigsäure versetzt und 10 min gerührt, wobei ein großer Teil des Eduktes in Lösung geht. Dann gibt man 85.93 g (0.613 mol) 2-Fluorbenzylhydrazin hinzu und kocht über Nacht. Nach Abkühlen werden die ausgefallenen Kristalle des Natriumtrifluoracetats abgesaugt, mit Dioxan gewaschen und die Lösung roh weiter umgesetzt.

### V b) 1-(2-Fluorbenzyl)-1H pyrazolo[3,4-b]pyridin-3-carbonsäureethylester

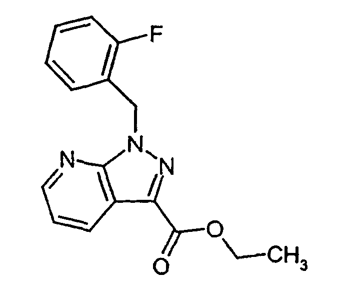

Die aus V a) erhaltene Lösung wird mit 61.25 ml (60.77 g, 0.613 mol) Dimethylaminoacrolein und 56.28 ml (83.88 g, 0.736 mol) Trifluoressigsäure versetzt und unter Argon 3 Tage lang gekocht. Anschließend wird das Lösungsmittel im Vakuum verdampft, der Rückstand in 21 Wasser gegeben und dreimal mit je 11 Essigester extrahiert. Die vereinigten organischen Phasen werden mit Magnesiumsulfat getrocknet und einrotiert. Man chromatographiert auf 2.5 kg Kieselgel und eluiert mit einem Toluol/Toluol-Essigester = 4:1 -Gradienten. Ausbeute: 91.6 g (49.9 % d.Th. über zwei Stufen).
Smp. 85°C
R_{f} (SiO₂, T1E1): 0.83

### V c) 1-(2-Fluorbenzyl)-1H-pyrazolo[3,4-b]pyridin-3-carboxamid

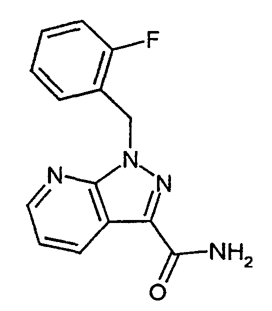

10.18 g (34 mmol) des in Beispiel V b) erhaltenen Esters werden in 150 ml mit Ammoniak bei 0 - 10°C gesättigtem Methanol vorgelegt. Man rührt zwei Tage bei Raumtemperatur und engt anschließend im Vakuum ein.
R_{f} (SiO₂, T1E1): 0.33

### V d) 3-Cyano-1-(2-fluorbenzyl)-1H-pyrazolo[3,4-b]pyridin

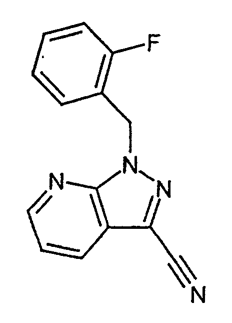

36.1 g (133 mmol) 1-(2-Fluorbenzyl)-1H-pyrazolo[3,4-b]pyridin-3-carboxamid aus Beispiel V c) werden in 330 ml THF gelöst und mit 27 g (341 mmol) Pyridin versetzt. Anschließend gibt man innerhalb von 10 min 47.76 ml (71.66 g, 341 mmol) Trifluoressigsäureanhydrid hinzu, wobei die Temperatur bis auf 40 °C ansteigt. Man rührt über Nacht bei Raumtemperatur. Anschließend wird der Ansatz in 11 Wasser gegeben und dreimal mit je 0.5 1 Essigester extrahiert. Die organische Phase wird mit gesättigter Natriumhydrogencarbonatlösung und mit 1 N HCl gewaschen, mit MgSO₄ getrocknet und einrotiert.
Ausbeute: 33.7 g (100 % d.Th.)
Smp: 81°C
R_{f} (SiO₂, T1E1): 0.74

### V e) (2-Fluorbenzyl)-1H-pyrazolo[3, 4-b]pyridin-3-carboximidsäuremethylester

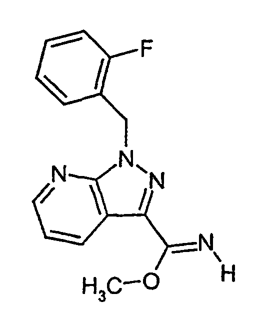

Man löst 30.37 g (562 mmol) Natriummethylat in 1.5 1 Methanol und gibt 36.45 g (144.5 mmol) 3-Cyano-1-(2-fluorbenzyl)-1H-pyrazolo[3,4-b]pyridin (aus Beispiel V d) hinzu. Man rührt 2 Stunden bei Raumtemperatur und setzt die erhaltene Lösung direkt für die nächste Stufe ein.

### V f) 1-(2-Fluorbenzyl)1H-pyrazolo[3,4-b]pyridin-3-carboxamidin

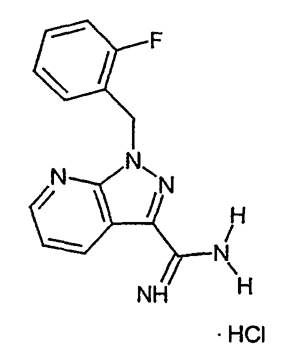

Die aus Beispiel V e) erhaltene Lösung von (2-Fluorbenzyl)-1H-pyrazolo[3,4-b]pyridin-3-carboximidsäuremethylester in Methanol wird mit 33.76 g (32.19 ml, 562 mmol) Eisessig und 9.28 g (173 mmol) Ammoniumchlorid versetzt und über Nacht unter Rückfluss gerührt. Man verdampft das Lösungsmittel im Vakuum, verreibt den Rückstand gut mit Aceton und saugt den ausgefallenen Feststoff ab.
¹H-NMR (d₆-DMSO, 200 MHz): δ= 5,93 (s, 2H); 7,1-7,5 (m, 4 H); 7,55 (dd, 1H); 8,12 (dd, 1H); 8,30 (dd, 1H); 9,5 (bs, 4H-austauschbar) ppm.
MS (EI): m/z = 270,2 (M-HCl)

### VI. Synthese von 2-Cyano-2-(1,3-dioxo-1,3-dihydro-2H-isoindol-2-yl)ethenyl acetat

### VI a) (1,3-dioxo-1,3-dihydro-2H-isoindol-2-yl)acetonitril

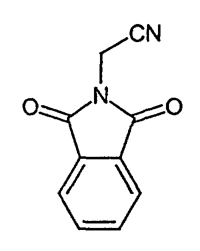

Phthalimid (1,35 g, 9.17 mmol, 1.1 Äquiv.) und Kaliumcarbonat (5,76 g, 41.7 mmol, 5.0 Äquiv.) wurden in DMF suspendiert und nach 10 min mit Bromacetonitril (1.00 g, 8.34 mmol) versetzt. Man rührte für 2 h bei Raumtemperatur und filtrierte anschließend vom ausgefallenen Niederschlag. Die Mutterlauge wurde mit EE verdünnt und gegen gesättigte wässrige NaCl-Lösung extrahiert. Die vereinigten organischen Phasen wurden am Rotationsverdampfer eingeengt. Auf diese Weise erhielt man 1.48 g (95 %) der gewünschten Verbindung.
- Ausbeute:: 1.48 g (95 %)
- R_{f}-Wert:: 0.76 (CH₂Cl₂/MeOH 100/1)
- ¹H-NMR:: (200 MHz, D₆-DMSO), δ = 4.75 (s, 2 H, CH₂), 7.8-8.0 (m, 4 H, Ar-H)
- MS:: (DCI), *m*/*z* = 204 ([M+NH₄]⁺), 221 ([M+N₂H₇]⁺), 390 ([2M+NH₄]⁺)
- LCMS:: Retentionszeit: 2.8 min (Säule: Symmetry, C-18, 3.5 µm, 50X2.1 mm, Fluss 0.5 ml/min, 40°C, Gradient: Wasser (+0.1 % Ameisensäure):Acetonitril (+0.1 % Ameisensäure) bei 0 min: 90:10, bei 7.5 min 10:90)); MS: (ESI pos.), *m*/*z* = keine Ionisation, (ESI neg.), *m*/*z* = keine Ionisation

### VI b) 2-Cyano-2-(1,3-dioxo-1,3-dihydro-2H isoindol-2-yl)ethenyl acetat (E,Z-Gemisch)

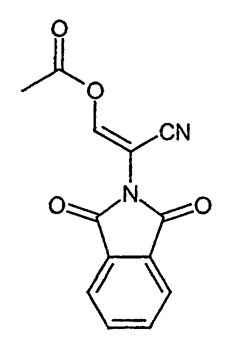

KOtBu (379 mg, 3.55 mmol, 2.2 Äquivalente) wurde in THF suspendiert und innerhalb von 10 min mit einer Lösung von (1,3-Dioxo-1,3-dihydro-2H-isoindol-2-yl)acetonitril (0.30 g, 1.6 mmol) aus Bsp. VI a) und Ameisensäureethylester (0.29 ml, 0.26 g, 3.6 mmol, 2.2 Äquivalente) in THF versetzt. Nach 2 h bei Raumtemperatur kühlte man auf 0°C und versetzte mit einer Lösung aus Acetanhydrid (0.21 ml, 0.23 g, 2.3 mmol, 1.4 Äquivalente) und Essigsäure (0.18 ml, 0.19 g, 3.2 mmol, 2 Äquivalente). Man ließ auf Raumtemperatur erwärmen, rührte für 1 h nach und versetzte mit EE und H₂O. Nach Phasentrennung wurden die organischen Phasen vereinigt und am Rotationsverdampfer eingeengt.
- Ausbeute:: 0.36 g (88 %)
- R_{f}-Wert:: 0.58 (CH₂Cl₂/MeOH 5/1)
- MS:: (DCI), *m*/*z* = 274 ([M+NH₄]⁺)
- LCMS:: Ret.-zeit: 2.9 min (Säule: Symmetry, C-18, 3.5 µm, 50X2.1 mm, Fluss 0.5 ml/min, 40°C, Gradient: Wasser (+0.1 % Ameisensäure):Acetonitril (+0.1 % Ameisensäure) bei 0 min: 90:10, bei 7.5 min 10:90)); MS: (ESI pos.), *m*/*z* = 256 ([M+H]⁺)

### VIIa. Synthese von 2-Cyano-2-(2-oxo-1,3-oxazolidin-3-yl)ethenyl-acetat (E/Z-Gemisch)

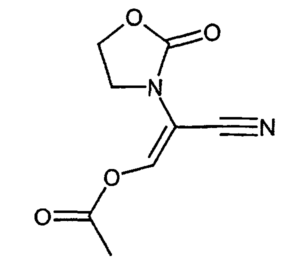

Eine Lösung aus 4.10 g (32.5 mmol) (2-Oxo-1,3-oxazolidin-3-yl)acetonitril aus Bsp. IV a und 5.30 g (71.5 mmol) Ameisensäureethylester in 20 ml wasserfreiem THF wurde unter Eiskühlung in eine Suspension aus 8.03 g (71.5 mmol) Kalium-*tert*-butanolat in 40 ml wasserfreiem THF getropft. Die Mischung wurde anschließend 1 h bei Raumtemperatur gerührt. Anschließend tropfte man unter Eiskühlung eine Lösung aus 4.65 g (4.29 ml = 45.5 mmol) Acetanhydrid in 4.30 g (4.09 ml = 71.5 mmol) Essigsäure zu und rührte 40 Minuten bei Raumtemperatur. Die Mischung wurde über eine kurze Kieselgel-Fritte mit Dichlormethan/Methanol als Eluens filtriert. Das Eluens wurde über Natriumsulfat getrocknet und am Rotationsverdampfer bei 40°C zur Trockne eingeengt. Das in 57proz. Ausbeute entstandene Rohprodukt wurde direkt in die nächste Reaktion eingesetzt.

Analog wurde hergestellt.

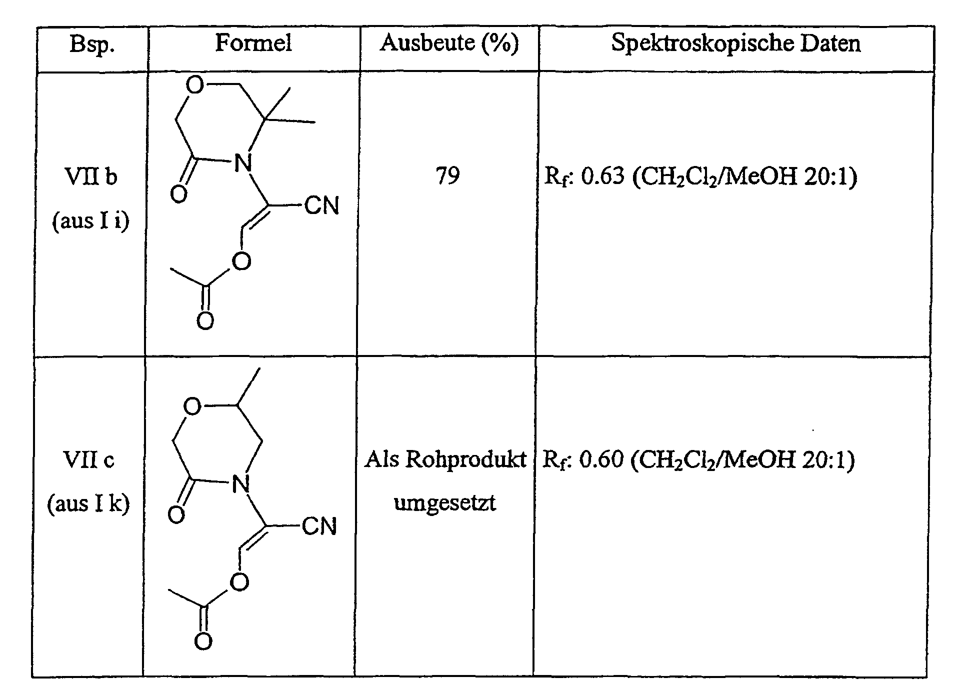

### VIII. Synthese von 2-Cyano-2-(2-oxo-1,3-thiazolidin-3-yl)ethenyl-acetat (E/Z-Gemisch)

### VIII a) (2-Oxo-1,3-thiazolidin-3-yl)acetonitril

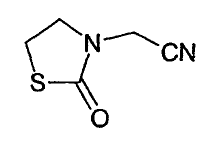

Die Herstellung erfolgte analog zu Beispiel IV a aus Bromacetonitril und 2-Thiazolidinon. Die Substanz wurde als DMF-haltiges Rohprodukt in die nächste Reaktion eingesetzt.

### VIII b) 2-Cyano-2-(2-oxo-1,3-thiazolidin-3-yl)ethenyl-acetat (E/Z-Gemisch)

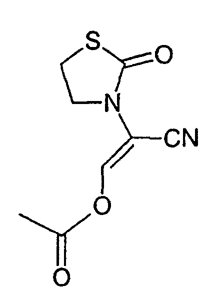

Die Herstellung erfolgte analog zu Beispiel VII aus (2-Oxo-1,3-thiazolidin-3-yl)acetonitril aus Bsp. VIIIa, Ethylformiat und Acetanhydrid. Die Substanz wurde als Rohprodukt in die nächste Reaktion eingesetzt.

### IX. Synthese von (2E/Z)-3-(Dimethylamino)-2-(3,3,4-trimethyl-2,5-dioxo-1-pyrrolidinyl)-2-propennitril

### IX a) (3,3,4-Trimethyl-2,5-dioxo-1-pyrrolidinyl)acetonitril

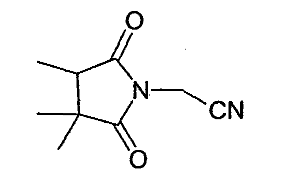

Die Synthese erfolgte analog zu Beispiel VI a aus (3,3,4-Trimethyl-2,5-dioxo-1-pyrrolidin (erhältlich aus Trimethylbernsteinsäure und Ammoniak nach Auwers; Oswald; Justus Liebigs Ann. Chem.; 285; 1895; 307; Trimethylbernsteinsäure kann aus 2-Bornanon durch Umsetzung mit Salpetersäure gewonnen werden nach Bredt; Chem.Ber.; 27; 1894; 2093) und Bromacetonitril mit der Ausnahme, dass statt DMF Aceton als Lösungsmittel verwendet wurde. Die Substanz wurde als Rohprodukt in die nächste Reaktion eingesetzt.
- Ausbeute:: 99.9 %
- GC-MS:: Rₜ = 8.08 min (Methode H).
MS (TOF-CI), *m*/*z* = 181 ([M+H]⁺).

### IX b) (2E/Z)-3-(Dimethylamino)-2-(3,3,4-trimethyl-2,5-dioxo-1-pyrrolidinyl)-2-propennitril

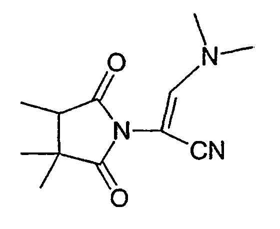

Die Synthese erfolgte analog zu Beispiel II a aus (3,3,4-Trimethyl-2,5-dioxo-1-pyrrolidinyl)acetonitril aus Bsp. IX a und tert-Butoxy-bis(dimethylamino)methan.
- Ausbeute:: 27 %
- LC-MS:: Rₜ = 2.02 min (Methode E).
MS (ESI pos.), *m*/*z* = 236 ([M+H]⁺), 471 ([2M+H]⁺).

### X. Synthese von 2-Cyano-2-(1-methyl-2,4-dioxo-3-azabicyclo[3.1.0]hex-3-yl)ethenyl acetat (E/Z-Gemisch)

### X a) (1-Methyl-2,4-dioxo-3-azabicyclo[3.1.0]hex-3-yl)acetonitril

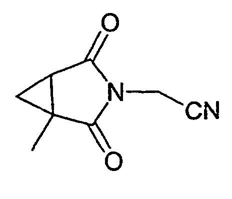

Die Synthese erfolgte analog zu Beispiel VI a aus 1-Methyl-3-azabicyclo[3.1.0]hexane-2,4-dion (erhältlich aus 3-Methyl-1H-pyrrol-2,5-dion durch Cyclopropanierung nach bekannten Verfahren, z. B. nach Annoura, H.; Fukunaga, A.; Uesugi, M.; Tasuoka, T.; Horikawa, Y.; Bioorg Med Chem Lett 1996, 6, 763-766) und Bromacetonitril mit der Ausnahme, dass statt DMF Aceton als Lösungsmittel verwendet wurde. Die Substanz wurde als Rohprodukt in die nächste Reaktion eingesetzt.
- Ausbeute:: 99.8 %
- R_{f}:: 0.52 (CH₂Cl₂/MeOH 20:1)

### X b) 2-Cyano-2-(1-methyl-2,4-dioxo-3-azabicyclo[3.1.0]hex-3-yl)ethenyl-acetat (E/Z-Gemisch)

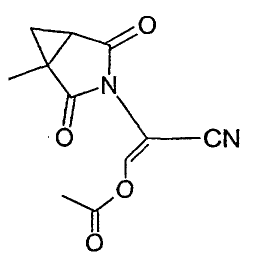

Die Synthese erfolgte analog zu Beispiel VII aus (1-Methyl-2,4-dioxo-3-azabicyclo[3.1.0]hex-3-yl)acetonitril aus Bsp. X a und Ameisensäureethylester, halium-*tert*-butanolat sowie Acetanhydrid/Essigsäure. Die Substanz wurde als Rohprodukt in die nächste Reaktion eingesetzt.
- Ausbeute:: 65 %

### XI. Synthese von (E/Z)-2-Cyano-2-(3,5-dioxo-10-oxa-4-azatricyclo[5.2.1.0^{2,6}]-dec-4-yl)ethenyl-acetat

### XI a) (3,5-Dioxo-10-oxa-4-azatricyclo[5.2.1.0^{2,6}]dec-4-yl)acetonitril

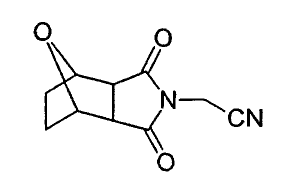

Die Synthese erfolgte analog zu Beispiel VI a aus 10-Oxa-4-azatricyclo[5.2.1.02,6]decan-3,5-dion (erhältlich aus Maleinimid und Furan durch Diels-Alder-Reaktion z. B. nach Padwa, A.; Dimitroff, M.; Waterson, A. G.; Wu, T.; J Org Chem 1997, 62, 4088-4096 und nachfolgender Hydrierung nach Ansell, M. F.; Caton, M. P. L.; North, P. C.; Tetrahedron Lett. 1982, 23, 2811) und Bromacetonitril mit der Ausnahme, dass statt DMF Aceton als Lösungsmittel verwendet wurde. Die Substanz wurde als Rohprodukt in die nächste Reaktion eingesetzt.
- Ausbeute:: 15 %
- GC-MS:: Rₜ = 12.05 min (Methode H).
MS (TOF-CI), *m*/*z* = 224 ([M+NH₄]⁺).

### XI b) (E/Z)-2-Cyano-2-(3,5-dioxo-10-oxa-4-azatricyclo[5.2.1.0^{2,6}]dec-4-yl)ethenylacetat

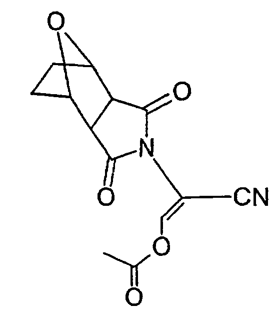

Die Synthese erfolgte analog zu Beispiel VII aus (3,5-Dioxo-10-oxa-4-azatricyclo[5.2.1.0^{2,6}]dec-4-yl)acetonitril aus Bsp. XI a und Ameisensäureethylester, Kalium-*tert*-butanolat sowie Acetanhydrid/Essigsäure. Die Substanz wurde als Rohprodukt in die nächste Reaktion eingesetzt.
- Ausbeute:: 61 %
- R_{f}:: 0,75 (CH₂Cl₂/MeOH 20:1)

### Beispiele

### 1. 4-{4-Amino-2-[1-(2-fluorobenzyl)-1H-pyrazolo[3,4-b]pyridin-3-yl]-5-pyrimidinyl}-3-morpholinon

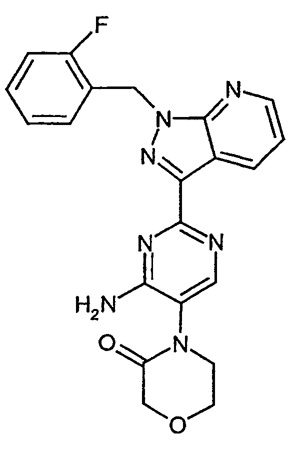

1.50 g (7.68 mmol) (2)-3-(Dimethylamino)-2-(3-oxo-4-morpholinyl)-2-propionitril (E/Z-Gemisch) aus Beispiel II a und 2.48 g ( 9.22 mmol) 1-(2-Fluorobenzyl)-1Hpyrazolo[3,4-b]pyridin-3-carboximidamid aus Beispiel V wurden in 90 ml Xylol gelöst und bei 120°C über Nacht gerührt. Die Mischung wird über Kieselgel mit Dichlormethan/Methanol (Gradient 200:1/50:1/20:1) chromatographiert.
- Ausbeute.:: 200 mg (5.65 %)
- Rf-Wert:: 0.50 (Dichlormethan/Methanol 10:1)
- ¹H-NMR:: (300 MHz, D₆-DMSO), δ = 3.51-3.60 (br.s, 2H, (CH₂N), 3.98-4.06 (br. t, 2H, CH₂O), 4.20 (s, 2H, CH₂O), 5.83 (s, 2H, CH₂), 7.10-7.41 (m, 7H, Ar-H und NH₂), 8.24 (s, 1H, Pyrimidin-H), 8.66 (dd, 1H, Pyridin-H), 8.96 (dd, 1H, Pyridin-H)
- MS:: (ESI pos.), *m*/*z* = 420 ([M+H]⁺)

Auf analoge Weise wurden hergestellt:

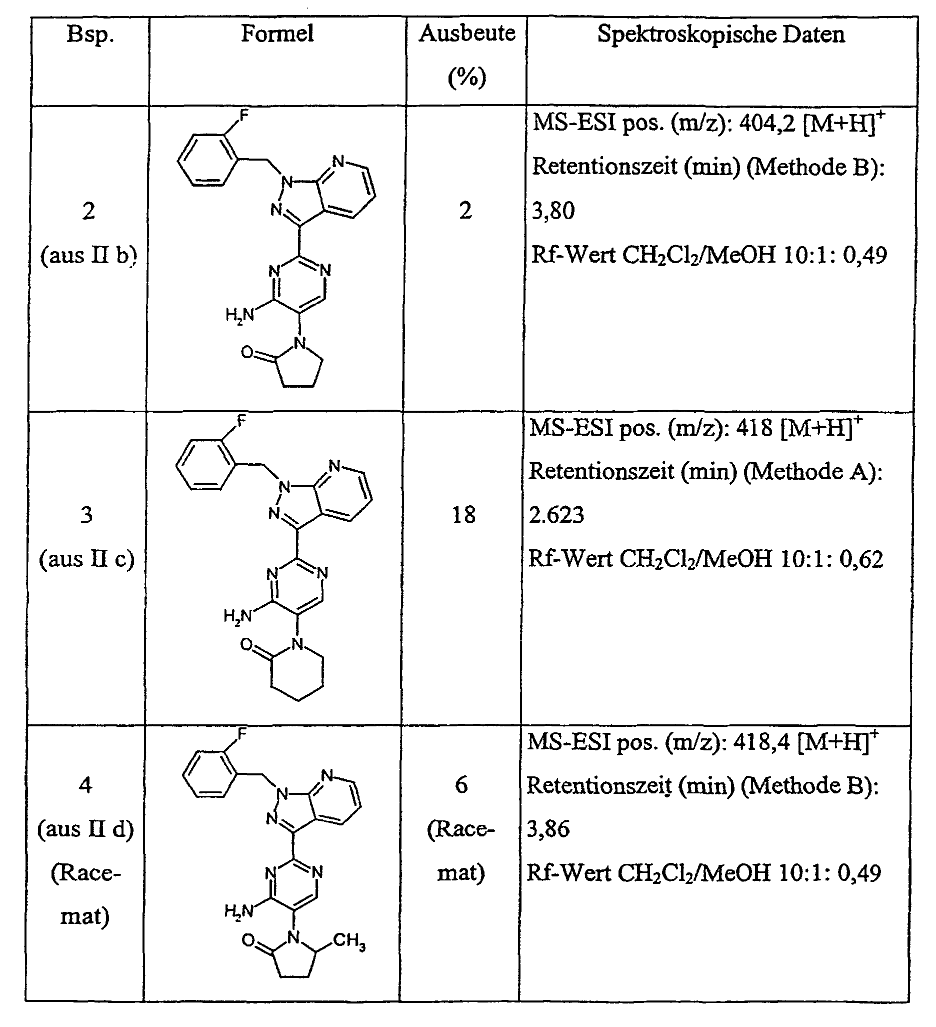

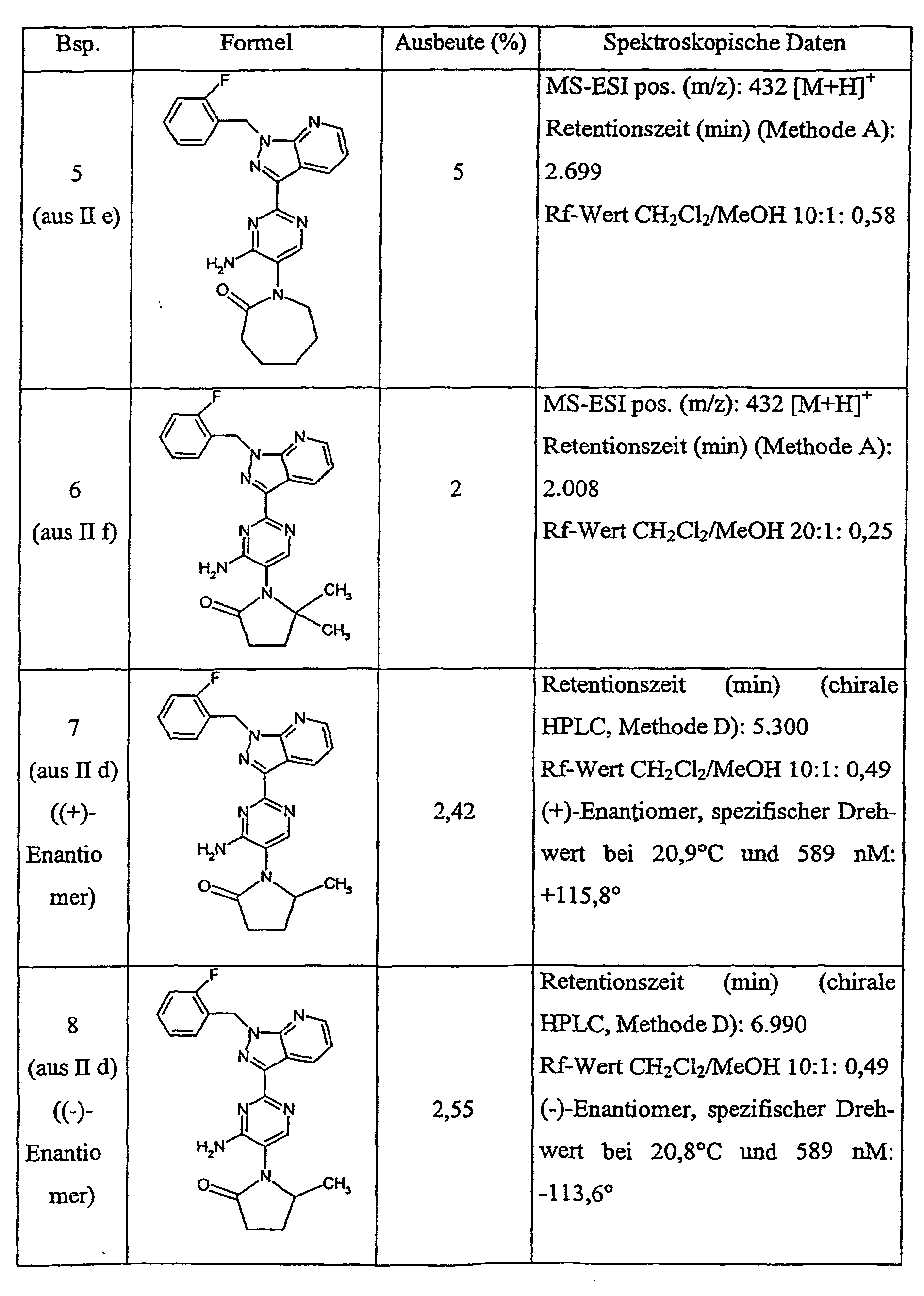

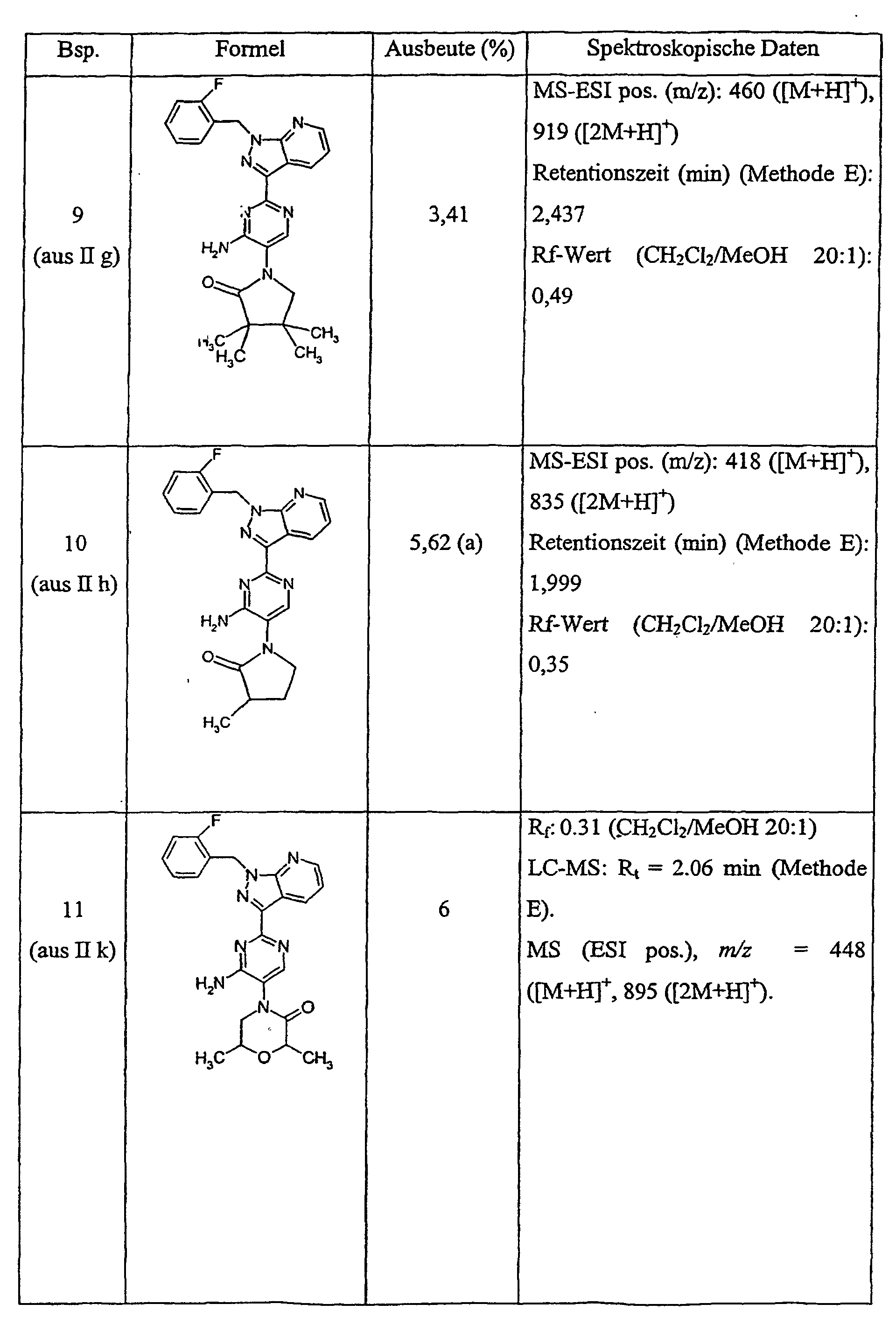

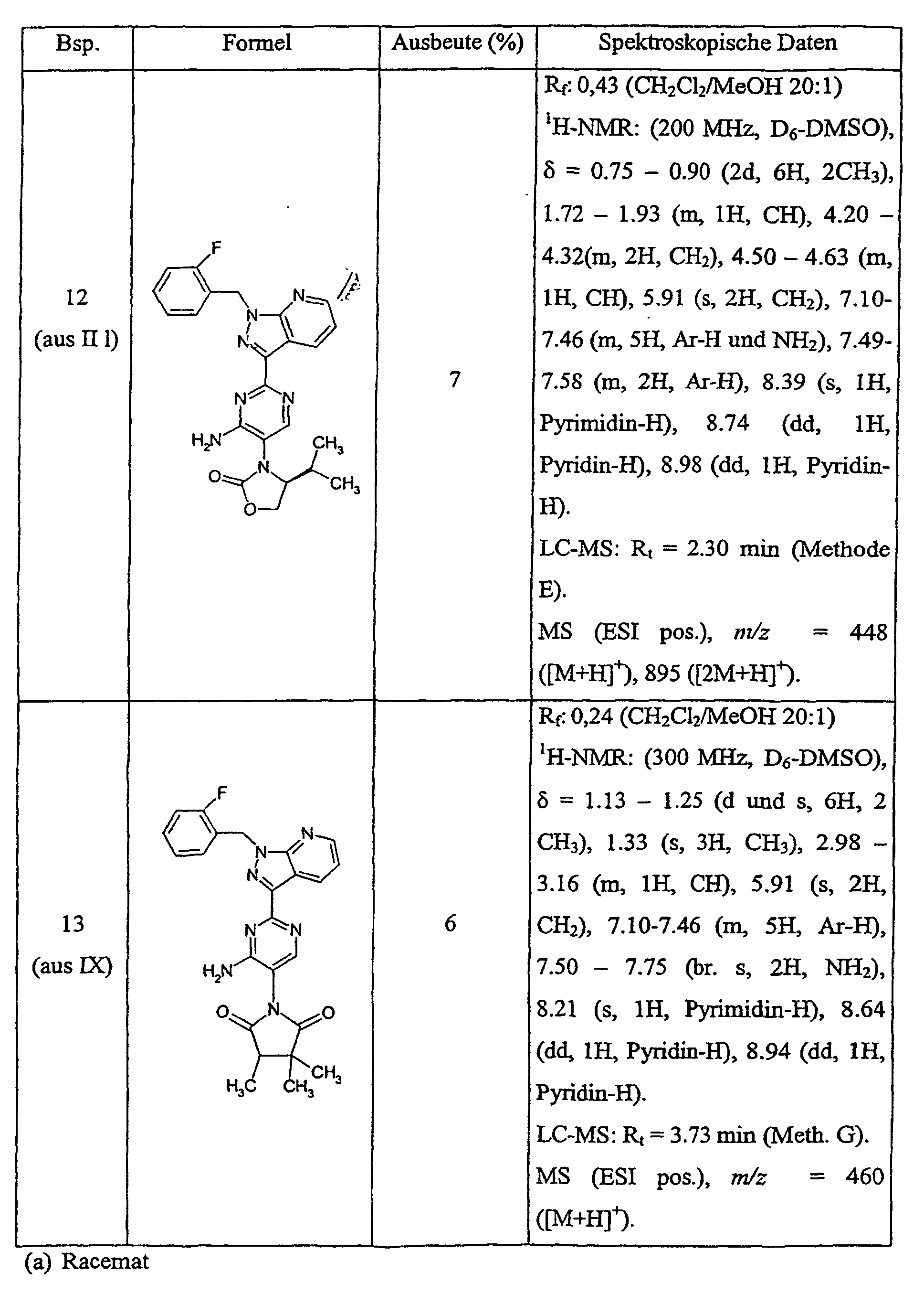

### 14. 3-{4-Amino-2-[1-(2-fluorobenzyl)-1H-pyrazolo[3,4-b]pyridin-3 yl]-5-pyrimidinyl}-5,5-dimethyl-1,3-oxazolidin-2,4-dion

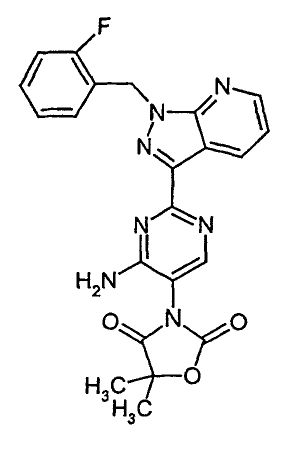

2.30 g (10.3 mmol) (2)-3-(Dimethylamino)-2-(5,5-dimethyl-2,4-dioxo-1,3-oxazolidin-3-yl)-2-propionitril (E/Z-Gemisch) aus Beispiel III und 1.39 g ( 5.15 mmol) 1-(2-Fluorobenzyl)-1H-pyrazolo[3,4-b]pyridin-3-carboximidamid aus Beispiel V wurden in 90 ml Xylol gelöst und bei 120°C über Nacht gerührt. Die Mischung wurde zweimal über Kieselgel mit Dichlormethan/Methanol (1.Gradient 200:1/50:1/20:1, 2.Gradient 100:1/50:1) chromatographiert und anschließend durch präparative HPLC (Säule: Kromasil 100 C 18 5 µm 250x20 mm Nr. 101131R, Fluss: 25 ml/min, Temp. 50°C, Gradient Wasser/Acetonitril bei 0 min: 55/45, bei 14 min 55/45) weiter aufgereinigt.
- Ausbeute:: 33.9 g (1.47%)
- ¹H-NMR:: (300 MHz, D₆-DMSO), δ =1.69 (s, 6H, CH₃), 5.85 (s, 2H, CH₂), 7.10-7.26 (m, 3H, Ar-H), 7.30-7.42 (m, 2H, Ar-H), 7.59 (br.s, 2H, NH₂), 8.41 (s, 1H, Pyrimidin-H), 8.66 (dd, 1H, Pyridin-H), 8.95 (dd, 1H, Pyridin-H)
- MS:: (ESI pos.), *m*/*z* = 448.4 ([M+H]⁺), 895.6 ([2M+H]⁺)

### 15. 3-{4-Amino-2-[1-(2-fluorobenzyl)-1H-pyrazolo [3,4-b] pyridin-3-yl]-5-pyrimidinyl}-1,3-oxazolidin-2-on

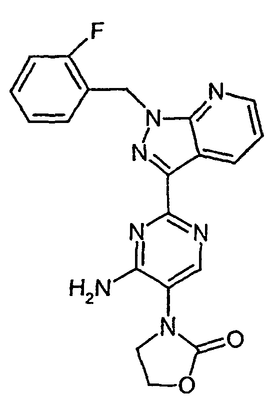

1.14 g (6.29 mmol) (2)-3-(Dimethylamino)-2-(2-oxo-1,3-oxazolidin-3-yl)-2-propionitril (E/Z-Gemisch) aus Beispiel IV und 1.69 g (6.29 mmol) 1-(2-Fluorobenzyl)-1Hpyrazolo[3,4-b]pyridin-3-carboximidamid aus Beispiel V wurden in 50 ml Xylol gelöst und bei 120°C über Nacht gerührt. Die Mischung wurde über Kieselgel mit Dichlormethan/Methanol (Gradient 100:1/50:1) chromatographiert.
- Ausbeute:: 44.0 g (1.70 %)
- ¹H-NMR:: (200 MHz, D₆-DMSO), δ = 3.80 (t, 2H, CH₂N), 4.48 (t, 2H, CH₂O), 5.82 (s, 2H, CH₂), 7.10-7.42 (m, 7H, Ar-H und NH₂), 8.40 (s, 1H, Pyrimidin-H), 8.65 (dd, 1H, Pyridin-H), 8.95 (dd, 1H, Pyridin-H)
- MS:: (ESI pos.), *m*/*z* = 406 ([M+H]⁺), 811 ([2M+H]⁺)

Alternativ wurden 1.00 g (5.10 mmol) 2-cyano-2-(2-oxo-1,3-oxazolidin-3-yl)ethenylacetat (E/Z-Gemisch, ca. 10 % DMF enthaltend) aus Bsp. VII a und 1.06 g (3.92 mmol) 1-(2-Fluorobenzyl)-1H-pyrazolo[3,4-b]pyridin-3-carboximidamid aus Bsp. V wurden in 5 ml Toluol suspendiert und bei 120°C über Nacht gerührt. Nach dreitägigem Stehen bei Raumtemperatur wurde die Mischung mit Ethylacetat versetzt und anschließend am Rotationsverdampfer eingeengt. Der Rückstand wurde mit Dichlormethan/Methanol 10:1 über eine kurze Kieselgel-Fritte fiiltriert. Das Filtrat wurde am Rotationsverdampfer eingeengt und der Rückstand in CH₂Cl₂ aufgenommen. Die Lösung wurde über Celite filtriert. Nach dem Einengen des Filtrats wurde das Produkt in Form eines hellbeigenen Feststoffs erhalten. Die spektroskopischen Daten (1H-NMR, IC-MS-ESI) der so erhaltenen Substanz sind identisch mit der zuvor aus dem Enamin (Beispiel 4B) hergestellten Verbindung.
- Ausbeute:: 629 mg (39.6 %)

Die Reaktion kann mit gleichem Erfolg auch in Xylol bei 120-140°C oder in Essigsäure bei 120°C durchgeführt werden. Auch ein Zusatz von Lewis-Säuren ist möglich, z.B. Zink(II)acetat, Scandium(III)trifluormethansulfonat, Mangan(II)acetat, Cobalt(II)acetat, Yttrium(III)trifluormethansulfonat, Bortrifluorid-Diethylether-Komplex. Die Reinigung kann auch chromatographisch an Kieselgel (Dichlormethan/Methanol (Gradient 30:1/20:1)) mit gegebenenfalls anschließender Reinigung durch präparative HPLC (Methode G) erfolgen.

Analog zu diesen alternativen Herstellungsverfahren wurden die folgenden Verbindungen hergestellt:

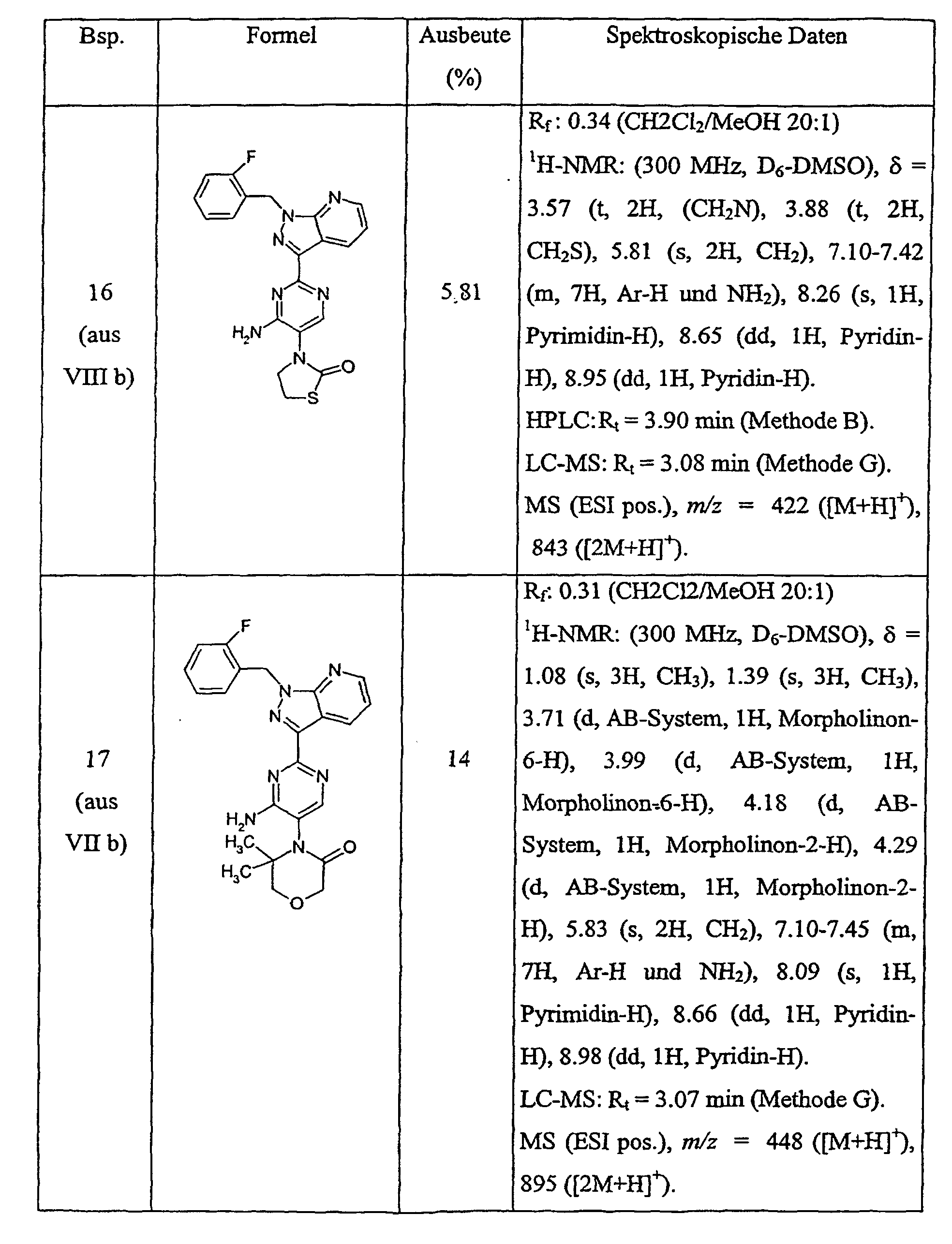

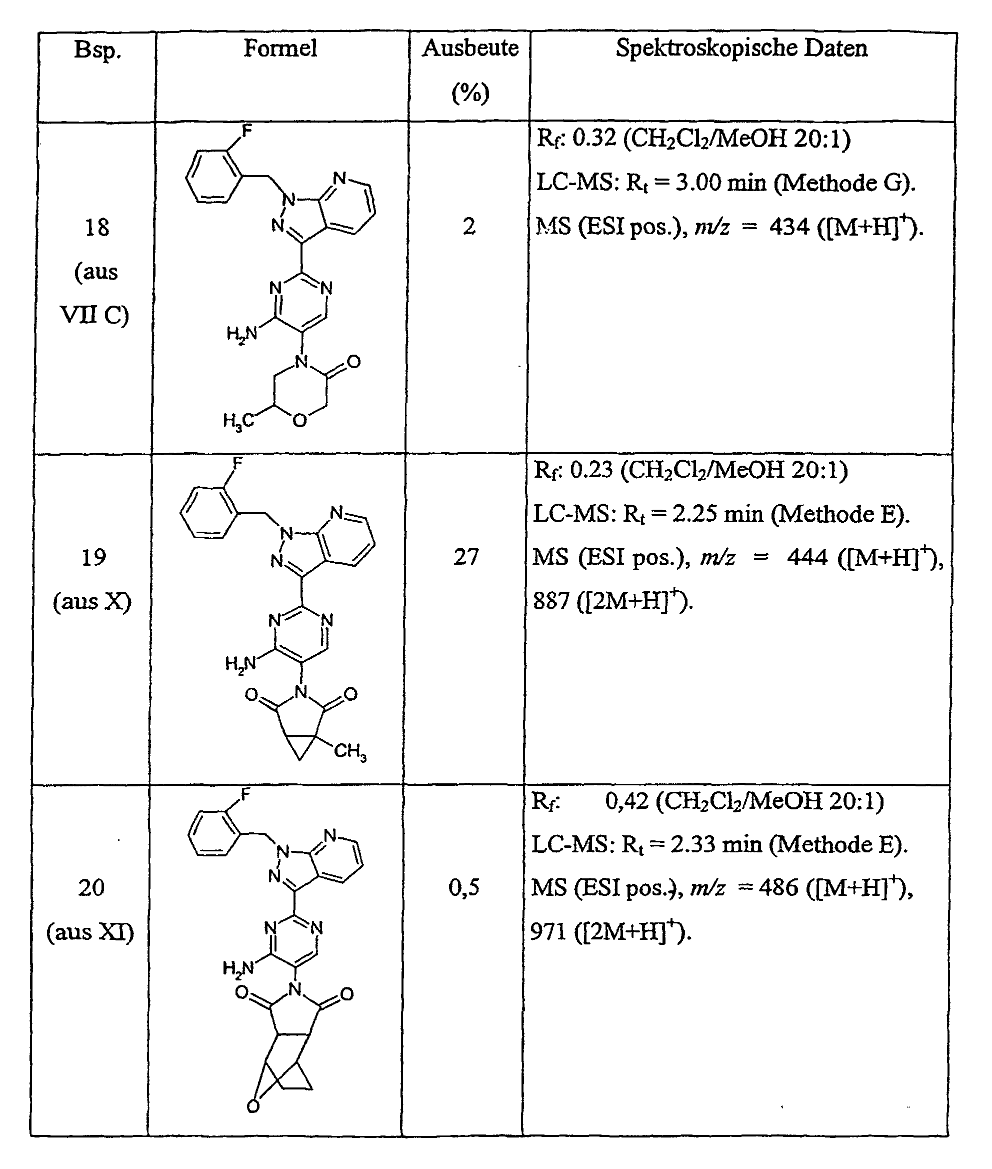

### 21. N-[2-[1-(2-Fluorobenzyl)-1H-pyrazolo[3,4-b]pyridin-3-yl]-5-(2-oxo-1,3-oxazolidin-3-yl)-4-pyrimidinyl]acetamid

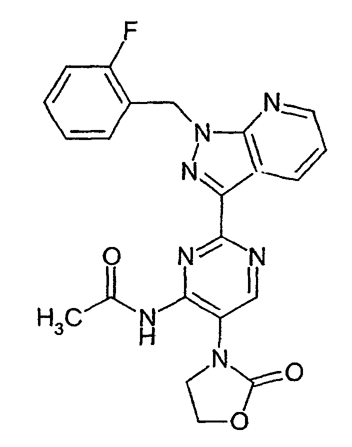

Die Synthese erfolgte nach der Alternativ-Herstellungsvorschrift zu Beisp. 15 aus 2-Cyano-2-(2-oxo-1,3-oxazolidin-3-yl)ethenyl-acetat (E/Z-Gemisch) aus Bsp. VII a und 1-(2-Fluorobenzyl)-1H-pyrazolo[3,4-b]pyridin-3-carboximidamid aus Bsp. V durch 4-tägiges Rühren in Essigsäure bei 125°C. Die Reinigung erfolgte durch präparative HPLC (Methode F) Das so erhaltene Produkt wurde, mit Ethylacetat aufgeschlämmt, abgesaugt und mit Diethylether nachgewaschen.
- Ausbeute:: 4 %
- R_{f}: 0,49 (CH₂Cl₂/MeOH 20:1)
- ¹H-NMR:: (300 MHz, D₆-DMSO), δ = 1.91 (s, 3H, COCH₃), 4.13 (t, 2H, CH₂N), 4.32 (t, 2H, CH₂O), 5.82 (s, 2H, CH₂), 7.10-7.44 (m, 5H, Ar-H), 8.58 (s, 1H, Pyrimidin-H), 8.64 (dd, 1H, Pyridin-H), 8.88 (dd, 1H, Pyridin-H), 12.18 - 12.32 (br. s, 1H, NHCO).
- LC-MS:: Rₜ = 3.73 min (Methode E).
MS (ESI pos.), *m*/*z* = 448 ([M+H]⁺, 895 ([2M+H]⁺).

### 22. 2-{4-amino-2-[1-(2-fluorobenzyl)-1H-pyrazolo[3,4-b]pyridin-3-yl]-5-pyrimidinyl}-1H-isoindol-1,3(2H)-dion

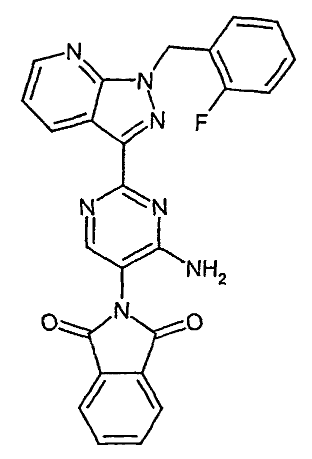

1-(2-Fluorobenzyl)-1H-pyrazolo[3,4-b]pyridin-3-carboximidamid (0.325 g, 1.06 mmol) aus Bsp. V, 2-Cyano-2-(1,3-dioxo-1,3-dihydro-2H-isoindol-2-yl)ethenylacetat (*E*,*Z*-Gemisch, 0.300 g, 1.17 mmol, 1.1 Äquivalente) aus Bsp. VI und Triethylamin (0.30 ml, 0.22 g, 2.1 mmol, 2.0 Äquiv.) wurden in Toluol suspendiert und für 9 h unter Rückfluss erhitzt. Man ließ auf Raumtemperatur abkühlen, versetzte mit EE und filtrierte vom ausgefallenen Niederschlag. Der Niederschlag wurde per präparativer HPLC gereinigt (Säule: Cromsil 120 ODS, C-18, 10 µm, 250x30 mm, Fluss 50 ml/min, Raumtemperatur, Gradient: Wasser Acetonitril bei 0 min: 90: 10, bei 28 min 5:95).
- Ausbeute:: 0.027 g (5 %)
- ¹H-NMR:: (400 MHz, D₆-DMSO), δ = 5.86 *(s,* 2H, OCH₂), 7.1-7.3 und 7.32-7.6 (m, 7 H, Ar-H, NH₂), 7.9-8.0 (m, 4 H, Ar-H), 8.32 (s, 1 H, Ar-H), 8.65 (d, 1 H, Ar-H), 8.98 (d, 1 H, Ar-H)
- LCMS:: Retentionszeit: 3.79 min (Säule: Symmetry, C-18, 3.5 µm, 50X2.1 mm, Fluss 0.5 ml/min, 40°C, Gradient: Wasser (+0.1 % Ameisensäure):Acetonitril (+0.1 % Ameisensäure) bei 0 min: 90:10, bei 7.5 min 10:90)); MS: (ESI pos.), *m*/*z* = 466 ([M+H]⁺), (ESI neg.), *m*/*z =* 464 ([M-H]⁺)

## Patentansprüche

1. Verbindungen der allgemeinen Formel (I) 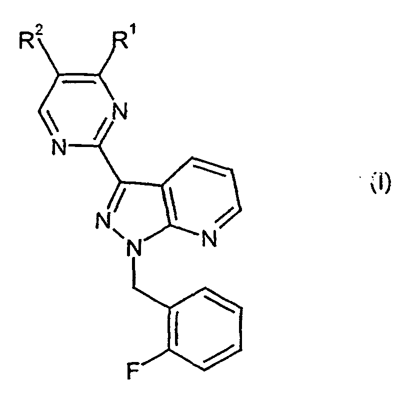 worin
R¹ für NH₂ oder für NHCO-C₁₋₆-alkyl steht;
R² für einen Rest der Formel R³NCOR⁴ steht, der über das Stickstoffatom an den Rest des Moleküls gebunden ist,
wobei
R³ und R⁴ zusammen mit der Amidgruppe, an die sie gebunden sind, einen fünf- bis siebengliedrigen Heterocyclus bilden, der gesättigt oder teilweise ungesättigt sein kann, gegebenenfalls ein weiteres Heteroatom aus der Gruppe N, O, S enthalten kann und ein bis fünf weitere Substituenten aus der Gruppe Oxo, C₁₋₆-Alkyl, Hydroxy, Hydroxy-C₁₋₆-alkyl, Halogen aufweisen kann beziehungsweise an einen C₆₋₁₀-Arylring oder an einen C₃₋₈-Cycloalkylring, bei welchem gegebenenfalls zwei Kohlenstoffatome über ein Sauerstoffatom miteinander verbunden sind, anelliert sein kann;
sowie Salze, Isomere und Hydrate davon.

2. Verbindungen nach Anspruch 1,
worin
R¹ für NH₂ oder für NHCO-C₁₋₆-alkyl steht;
R² für einen Rest der Formel R³NCOR⁴ steht, der über das Stickstoffatom an den Rest des Moleküls gebunden ist,
wobei
R³ und R⁴ zusammen mit der Amidgruppe, an die sie gebunden sind, einen gesättigten fünf- bis siebengliedrigen Heterocyclus bilden, der gegebenenfalls ein weiteres Heteroatom aus der Gruppe N, O, S enthalten kann und ein bis fünf weitere Substituenten aus der Gruppe Oxo, C₁₋₄-Alkyl, Hydroxy, Hydroxy-C₁₋₄-alkyl, Halogen aufweisen kann beziehungsweise an einen C₆₋₁₀-Arylring oder an einen C₃₋₈-Cycloalkylring, bei welchem gegebenenfalls zwei Kohlenstoffatome über ein Sauerstoffatom miteinander verbunden sind, anelliert sein kann;
sowie Salze, Isomere und Hydrate davon.

3. Verbindungen nach Anspruch 1,
worin
R¹ für NH₂ oder für NHCOCH₃ steht;
R² für einen Rest der Formel R³NCOR⁴ steht, der über das Stickstoffatom an den Rest des Moleküls gebunden ist,
wobei
R³ und R⁴ zusammen mit der Amidgruppe, an die sie gebunden sind, einen gesättigten fünf- bis siebengliedrigen Heterocyclus bilden, der gegebenenfalls ein weiteres Heteroatom aus der Gruppe N, O, S enthalten kann und ein bis fünf weitere Substituenten aus der Gruppe Oxo, C₁₋₄-Alkyl aufweisen kann beziehungsweise an einen Phenylring oder an einen C₃₋₈-Cycloalkylring, bei welchem gegebenenfalls zwei Kohlenstoffatome über ein Sauerstoffatom miteinander verbunden sind, anelliert sein kann;
sowie Salze, Isomere und Hydrate davon.

4. Verbindungen nach Anspruch 1,
worin
R¹ für NH₂ steht;
R² für einen Rest der Formel R³NCOR⁴ steht, der über das Stickstoffatom an den Rest des Moleküls gebunden ist,
wobei
R³ und R⁴ zusammen mit der Amidgruppe, an die sie gebunden sind, einen fünf bis siebengliedrigen Heterocyclus bilden, der gesättigt oder teilweise ungesättigt sein kann, gegebenenfalls ein weiteres Heteroatom aus der Gruppe N, O, S enthalten kann und ein bis fünf weitere Substituenten aus der Gruppe Oxo, C₁₋₆-Alkyl, Hydroxy, Hydroxy-C₁₋₆-alkyl, Halogen aufweisen kann beziehungsweise an einen C₆₋₁₀-Arylring anelliert sein kann;
sowie Salze, Isomere und Hydrate davon.

5. Verbindungen nach Anspruch 1,
worin
R¹ für NH₂ steht;
R² für einen Rest der Formel R³NCOR⁴ steht, der über das Stickstoffatom an den Rest des Moleküls gebunden ist,
wobei
R³ und R⁴ zusammen mit der Amidgruppe, an die sie gebunden sind, einen gesättigten fünf- bis siebengliedrigen Heterocyclus bilden, gegebenenfalls ein weiteres Sauerstoffatom enthalten kann und ein bis fünf weitere Substituenten aus der Gruppe Oxo, C₁₋₄-Alkyl, Hydroxy, Hydroxy-C₁₋₄-alkyl, F aufweisen kann beziehungsweise an einen C₆₋₁₀Arylring anelliert sein kann;
sowie Salze, Isomere und Hydrate davon.

6. Verbindungen nach Anspruch 1,
worin
R¹ für NH₂ steht;
R² für einen Rest der Formel R³NCOR⁴ steht, der über das Stickstoffatom an den Rest des Moleküls gebunden ist,
wobei
R³ und R⁴ zusammen mit der Amidgruppe, an die sie gebunden sind, einen fünf- oder sechsgliedrigen gesättigten Heterocyclus bilden, der gegebenenfalls ein weiteres Sauerstoffatom enthalten kann und ein bis fünf weitere Substituenten aus der Gruppe Oxo, C₁₋₄-Alkyl aufweisen kann beziehungsweise an einen Phenylring anelliert sein kann;
sowie Salze, Isomere und Hydrate davon.

7. Verfahren zur Herstellung von Verbindungen der Formel 1, umfassend
[A] die Umsetzung der Verbindung der Formel (II) 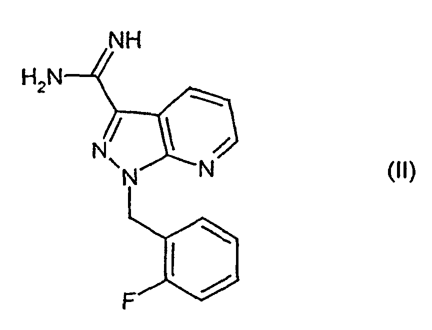 mit Verbindungen der Formel (III) 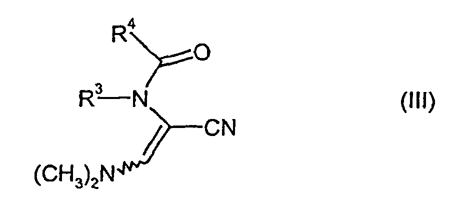 oder mit Verbindungen der Formel (IV) 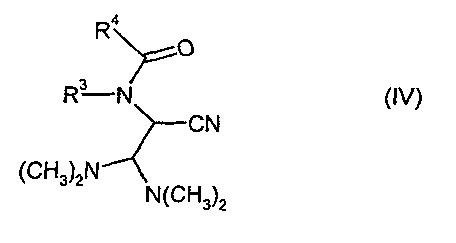 oder mit Verbindungen der Formel (V) 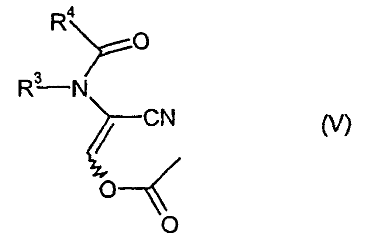 worin R³ und R⁴ die vorstehend angegebene Bedeutung haben,
in einem organischen Lösungsmittel gegebenenfalls in Gegenwart einer Base unter Erhitzen zu Verbindungen der Formel (I).

8. Verbindungen der allgemeinen Formel (I) zur Behandlung von Krankheiten.

9. Arzneimittel enthaltend mindestens eine Verbindung der allgemeinen Formel (I) gemäß Anspruch 1.

10. Verfahren zur Herstellung von Arzneimitteln, **dadurch gekennzeichnet, dass** man mindestens eine Verbindung der Formel (I) gemäß Anspruch 1, gegebenenfalls mit üblichen Hilfs- und Zusatzstoffen in eine geeignete Applikationsfonn überführt.

11. Arzneimittel enthaltend mindestens eine Verbindung der allgemeinen Formel (I) gemäß Anspruch 1 in Kombination mit organischen Nitraten oder NO-Donatoren.

12. Arzneimittel enthaltend mindestens eine Verbindung der allgemeinen Formel (I) gemäß Anspruch 1 in Kombination mit Verbindungen, die den Abbau von cyclischen Guanosinmonophosphat (cGMP) inhibieren.

13. Verwendung von Verbindungen der allgemeinen Formel (I) gemäß Anspruch 1 bei der Herstellung von Arzneimitteln zur Behandlung von Herz-Kreislauf-Erkrankungen.

14. Verwendung von Verbindungen der allgemeinen Formel (I) gemäß Anspruch 1 bei der Herstellung von Arzneimitteln zur Behandlung von Hypertonie.

15. Verwendung von Verbindungen der allgemeinen Formel (1) gemäß Anspruch 1 bei der Herstellung von Arzneimitteln zur Behandlung von thromboembolischen Erkrankungen und Ischämien.

16. Verwendung von Verbindungen der allgemeinen Formel (I) gemäß Anspruch 1 bei der Herstellung von Arzneimitteln zur Behandlung von sexueller Dysfunktion.

17. Verwendung von Verbindungen der allgemeinen Formel (I) gemäß Anspruch 1 bei der Herstellung von Arzneimitteln mit antiinflammatorischen Eigenschaften.

18. Verwendung von Verbindungen der allgemeinen Formel (I) gemäß Anspruch 1 bei der Herstellung von Arzneimitteln zur Behandlung von Erkrankungen des Zentralnervensystems.

19. Verwendung gemäß einem der Ansprüche 11 bis 16, wobei die Verbindungen der allgemeinen Formel gemäß Anspruch 1 in Kombination mit organischen Nitraten oder NO-Donatoren oder in Kombination mit Verbindungen, die den Abbau von cyclischen Guanosinmonophosphat (cGMP) inhibieren, eingesetzt werden.

## Claims

1. Compound of the formula (I) 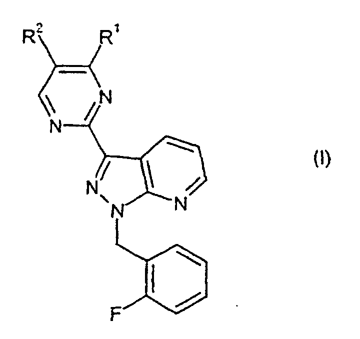 in which
R¹ is NH₂ or is NHCO-C₁₋₆-alkyl;
R² is a radical of the formula R³NCOR⁴ which is bonded via the nitrogen atom to the remainder of the molecule,
where
R³ and R⁴ together with the amide group to which they are bonded form a five- to seven-membered heterocycle which may be saturated or partially unsaturated, may optionally contain a further heteroatom from the group of N, O, S, and may have 1 to 5 further substituents from the group of oxo, C₁₋₆-alkyl, hydroxyl, hydroxy-C₁₋₆-alkyl, halogen, and may be fused to a C₆₋₁₀-aryl ring or to a C₃₋₈-cycloalkyl ring in which two carbon atoms are optionally connected together via an oxygen atom;
and salts, isomers and hydrates thereof.

2. Compound according to Claim 1,
in which
R¹ is NH₂ or is NHCO-C₁₋₆-alkyl;
R² is a radical of the formula R³NCOR⁴ which is bonded via the nitrogen atom to the remainder of the molecule,
where
R³ and R⁴ together with the amide group to which they are bonded form a saturated five- to seven-membered heterocycle which may optionally contain a further heteroatom from the group of N, O, S, and may have 1 to 5 further substituents from the group of oxo, C₁₋₄-alkyl, hydroxyl, hydroxy-C₁₋₄-alkyl, halogen, and may be fused to a C₆₋₁₀-aryl ring or to a C₃₋₈-cycloalkyl ring in which two carbon atoms are optionally connected together via an oxygen atom;
and salts, isomers and hydrates thereof.

3. Compound according to Claim 1,
in which
R¹ is NH₂ or is NHCOCH₃;
R² is a radical of the formula R³NCOR⁴ which is bonded via the nitrogen atom to the remainder of the molecule,
where
R³ and R⁴ together with the amide group to which they are bonded form a saturated five- to seven-membered heterocycle which may optionally contain a further heteroatom from the group ofN, O, S, and may have 1 to 5 further substituents from the group of oxo, C₁₋₄-alkyl, and may be fused to a phenyl ring or to a C₃₋₈-cycloalkyl ring in which optionally two carbon atoms are connected together via an oxygen atom;
and salts, isomers and hydrates thereof.

4. Compound according to Claim 1,
in which
R¹ is NH₂;
R² is a radical of the formula R³NCOR⁴ which is bonded via the nitrogen atom to the remainder of the molecule,
where
R³ and R⁴ together with the amide group to which they are bonded form a five- to seven-membered heterocycle which may be saturated or partially unsaturated, may optionally contain a further heteroatom from the group of N, O, S, and may have 1 to 5 further substituents from the group of oxo, C₁₋₆-alkyl, hydroxyl, hydroxy-C₁₋₆-alkyl, halogen, and may be fused to a C₆₋₁₀-aryl ring;
and salts, isomers and hydrates thereof.

5. Compound according to Claim 1,
in which
R¹ is NH₂;
R² is a radical of the formula R³NCOR⁴ which is bonded via the nitrogen atom to the remainder of the molecule,
where
R³ and R⁴ together with the amide group to which they are bonded form a saturated five- to seven-membered heterocycle [lacuna] may optionally contain a further oxygen atom and may have 1 to 5 further substituents from the group of oxo, C₁₋₄-alkyl, hydroxyl, hydroxy-C₁₋₄-alkyl, F, and may be fused to a C₆₋₁₀-aryl ring;
and salts, isomers and hydrates thereof.

6. Compound according to Claim 1,
in which
R¹ is NH₂;
R² is a radical of the formula R³NCOR⁴ which is bonded via the nitrogen atom to the remainder of the molecule,
where
R³ and R⁴ together with the amide group to which they are bonded form a five- or six-membered saturated heterocycle which may optionally contain a further oxygen atom and may have 1 to 5 further substituents from the group of oxo, C₁₋₄-alkyl, and may be fused to a phenyl ring;
and salts, isomers and hydrates thereof.

7. Process for preparing compounds of the formula 1, comprising
[A] reacting the compound of the formula (II) 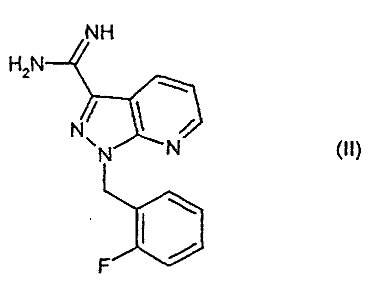 with compounds of the formula (III) 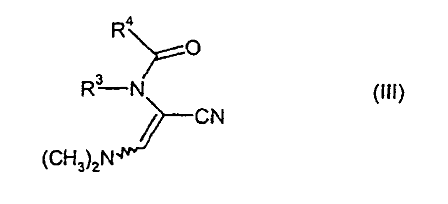 or with compounds of the formula (IV) 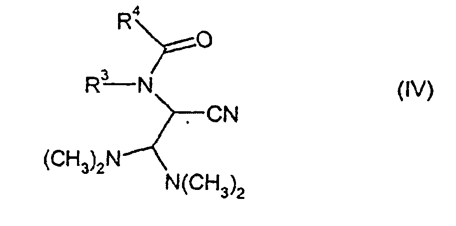 or with compounds of the formula (V) 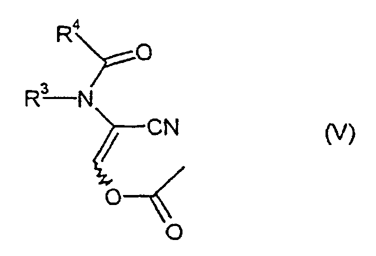 in which R³ and R⁴ have the meaning indicated above,
in an organic solvent where appropriate in the presence of a base with heating to give compounds of the formula (I).

8. Compound of the formula (I) for treating diseases.

9. Medicament comprising at least one compound of the formula (I) according to Claim 1.

10. Process for producing medicaments, **characterized in that** at least one compound of the formula (I) according to Claim 1 is converted where appropriate with conventional excipients and additives into a suitable administration form.

11. Medicament comprising at least one compound of the formula (I) according to Claim 1 in combination with organic nitrates or NO donors.

12. Medicament comprising at least one compound of the formula (I) according to Claim 1 in combination with compounds which inhibit the breakdown of cyclic guanosine monophosphate (cGMP).

13. Use of compounds of the general formula (I) according to Claim 1 for the production of medicaments for the treatment of cardiovascular disorders.

14. Use of compounds of the general formula (I) according to Claim 1 for the production of medicaments for the treatment of hypertension.

15. Use of compounds of the general formula (I) according to Claim 1 for the production of medicaments for the treatment of thromboembolic disorders and ischemias.

16. Use of compounds of the general formula (I) according to Claim 1 for the production of medicaments for the treatment of sexual dysfunction.

17. Use of compounds of the general formula (I) according to Claim 1 for the production of medicaments having antiinflammatory properties.

18. Use of compounds of the general formula (I) according to Claim 1 for the production of medicaments for the treatment of disorders of the central nervous system.

19. Use according to any of Claims 11 to 16, where the compounds of the general formula as claimed in claim 1 are employed in combination with organic nitrates or NO donors or in combination with compounds which inhibit the breakdown of cyclic guanosine monophosphate (cGMP).

## Revendications

1. Composés de formule générale (I) 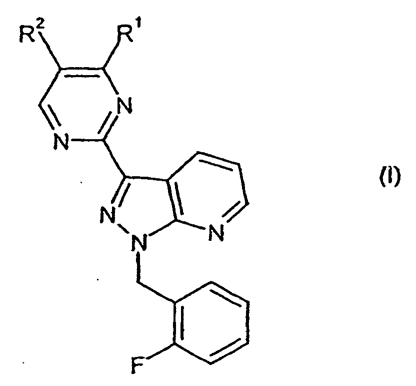 dans laquelle
R¹ est un groupe NH₂ ou un reste NHCO-(alkyle en C₁ à C₆) ;
R² est un reste de formule R³NCOR⁴ qui est lié par l'intermédiaire de l'atome d'azote au restant de la molécule,
dans lequel
R³ et R⁴ forment, conjointement avec le groupe amide auquel ils sont liés, un hétérocycle pentagonal à heptagonal qui peut être saturé ou partiellement insaturé, qui peut contenir éventuellement un autre hétéroatome du groupe N, O, S et qui peut porter un à cinq autres substituants du groupe oxo, alkyle en C₁ à C₆, hydroxy, hydroxy-(alkyle en C₁ à C₆), halogène, ou bien qui peut être condensé à un noyau aryle en C₆ à C₁₀ ou à un noyau cycloalkyle en C₃ à C₈ dans lequel, le cas échéant, deux atomes de carbone sont liés ensemble par l'intermédiaire d'un atome d'oxygène ;
ainsi que leurs sels, leurs isomères et leurs hydrates.

2. Composés suivant la revendication 1,
dans lesquels
R¹ est un groupe NH₂ ou un reste NHCO-(alkyle en C₁ à C₆) ;
R² est un reste de formule R³NCOR⁴ qui est liée par l'intermédiaire de l'atome d'azote au restant de la molécule,
dans lequel
R³ et R⁴ forment conjointement avec le groupe amide auquel ils sont liés un hétérocycle saturé pentagonal à heptagonal qui peut contenir éventuellement un autre hétéroatome du groupe N, O, S, et qui peut porter un à cinq autres substituants du groupe oxo, alkyle en C₁ à C₄, hydroxy, hydroxy-(alkyle en C₁ à C₄), halogéno, ou bien qui peut être condensé à un noyau aryle en C₆ à C₁₀ ou à un noyau cycloalkyle en C₃ à C₈, dans lequel le cas échéant deux atomes de carbone sont liés ensemble par l'intermédiaire d'un atome de carbone ;
ainsi que leurs sels, isomères et hydrates.

3. Composés suivant la revendication 1,
dans lesquels
R¹ est un groupe NH₂ ou un reste NHCOCH₃,
R² est un reste de formule R³NCOR⁴ qui est lié par l'intermédiaire d'un atome d'azote au restant de la molécule,
dans lequel
R³ et R⁴ forment conjointement avec le groupe amide auquel ils sont liés un hétérocycle saturé pentagonal à heptagonal, qui peut éventuellement contenir un autre hétéroatome du groupe N, O, S et qui peut porter 1 à 5 autres substituants du groupe oxo, alkyle en C₁ à C₄, ou bien qui peut être condensé à un noyau phényle ou à un noyau alkyle en C₃ à C₈ dans lequel, le cas échéant, deux atomes de carbone sont liés ensemble par l'intermédiaire d'un atome d'oxygène ;
ainsi que leurs sels, leurs isomères et leurs hydrates.

4. Composés suivant la revendication 1,
dans lesquels
R¹ est un groupe NH₂,
R² est un reste de formule R³NCOR⁴, qui est lié par l'intermédiaire de l'atome d'azote au restant de la molécule,
dans lequel
R³ et R⁴ forment conjointement avec le groupe amide auquel ils sont liés un hétérocycle pentagonal à heptagonal qui peut être saturé ou partiellement insaturé, qui peut contenir éventuellement un autre hétéroatome du groupe N, O, S et qui peut porter un à cinq autres substituants du groupe oxo, alkyle en C₁ à C₆, hydroxy, hydroxy-(alkyle en C₁ à C₆), halogéno, ou bien qui peut être condensé à un noyau aryle en C₆ à C₁₀ ;
ainsi que leurs sels, isomères et hydrates.

5. Composés suivant la revendication 1,
dans lesquels
R¹ est un groupe NH₂ ;
R² est un reste de formule R³NCOR⁴ qui est lié par l'intermédiaire de l'atome d'azote au restant de la molécule,
dans lequel
R³ et R⁴ forment, conjointement avec le groupe amide auquel ils sont liés, un hétérocycle saturé pentagonal à heptagonal, qui peut éventuellement contenir un autre atome d'oxygène et qui peut porter un à cinq autres substituants du groupe oxo, alkyle en C₁ à C₄, hydroxy, hydroxy-(alkyle en C₁ à C₄), F, ou bien qui peut être condensé à un noyau aryle en C₆ à C₁₀ ;
ainsi que leurs sels, leurs isomères et leurs hydrates.

6. Composés suivant la revendication 1,
dans lesquels
R¹ est un groupe NH₂ ;
R² est un reste de formule R³NCOR⁴ qui est lié par l'intermédiaire de l'atome d'azote au restant de la molécule,
dans lequel
R³ et R⁴ forment, conjointement avec le groupe amide auquel ils sont liés, un hétérocycle saturé pentagonal ou hexagonal qui peut éventuellement contenir un autre atome d'oxygène et qui peut porter un à cinq autres substituants du groupe oxo, alkyle en C₁ à C₄, ou bien qui peut être condensé à un noyau phényle ;
ainsi que leurs sels, leurs isomères et leurs hydrates.

7. Procédés de production de composés de formule (I), comprenant
[A] la réaction du composé de formule (II) 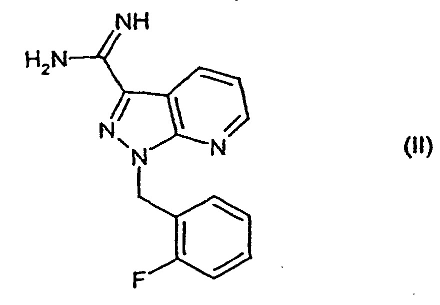 avec des composés de formule (III) 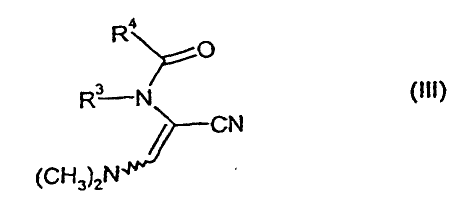 ou avec des composés de formule (IV) 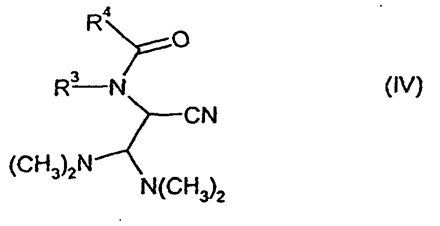 ou avec des composés de formule (V) 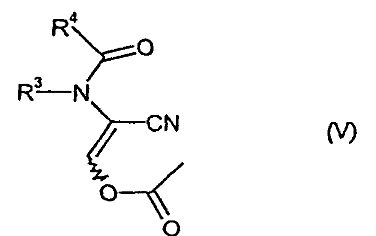 formules dans lesquelles R³ et R⁴ ont la définition indiquée ci-dessus,
dans un solvant organique, éventuellement en présence d'une base, en chauffant pour former des composés de formule (I).

8. Composés de formule (I), destinés au traitement de maladies.

9. Médicament contenant au moins un composé de formule générale (I) suivant la revendication 1.

10. Procédé de préparation de médicaments, **caractérisé en ce qu'**on fait prendre une forme convenable d'administration à au moins un composé de formule (I) suivant la revendication 1, éventuellement avec des substances auxiliaires et des additifs usuels.

11. Médicament contenant au moins un composé de formule générale (I) suivant la revendication 1, en association avec des nitrates organiques ou des donneurs de NO.

12. Médicament contenant au moins un composé de formule générale (I) suivant la revendication 1, en association avec des composés qui inhibent la dégradation de monophosphate de guanosine cyclique (GMP_{c}).

13. Utilisation de composés de formule générale (I) suivant la revendication 1 pour la préparation de médicaments destinés au traitement de maladies du système cardiovasculaire.

14. Utilisation de composés de formule générale (I) suivant la revendication 1 pour la préparation de médicaments destinés au traitement de l'hypertonie.

15. Utilisation de composés de formule générale (I) suivant la revendication 1 pour la préparation de médicaments destinés au traitement de maladies thromboemboliques et d'ischémies.

16. Utilisation de composés de formule générale (I) suivant la revendication 1 pour la préparation de médicaments destinés au traitement d'un dysfonctionnement sexuel.

17. Utilisation de composés de formule générale (I) suivant la revendication 1 pour la préparation de médicaments possédant des propriétés anti-inflammatoires.

18. Utilisation de composés de formule générale (I) suivant la revendication 1 pour la préparation de médicaments destinés au traitement de maladies du système nerveux central.

19. Utilisation suivant l'une des revendications 11 à 16, dans laquelle les composés de formule générale suivant la revendication 1 sont utilisés en association avec des nitrates organiques ou des donneurs de NO ou en association avec des composés qui inhibent la dégradation du monosphosphate de guanosine cyclique (GMP_{c}).
